(19) Europäisches Patentamt
European Patent Office
Office européen des brevets

(11) EP 4 119 164 A1

(12) **EUROPEAN PATENT APPLICATION**
published in accordance with Art. 153(4) EPC

(43) Date of publication:
**18.01.2023 Bulletin 2023/03**

(21) Application number: **21768762.3**

(22) Date of filing: **12.03.2021**

(51) International Patent Classification (IPC):
***A61K 45/00*** *(2006.01)* ***A61K 31/285*** *(2006.01)*
***A61K 31/555*** *(2006.01)* ***A61K 31/5377*** *(2006.01)*
***A61K 31/517*** *(2006.01)* ***A61P 31/14*** *(2006.01)*
***A61P 11/00*** *(2006.01)*

(52) Cooperative Patent Classification (CPC):
**A61K 31/285; A61K 31/517; A61K 31/5377; A61K 31/555; A61K 45/00; A61P 11/00; A61P 31/14**

(86) International application number:
**PCT/CN2021/080540**

(87) International publication number:
**WO 2021/180219 (16.09.2021 Gazette 2021/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.03.2020 CN 202010177429**

(71) Applicants:
- **Nuo-Beta Pharmaceutical Technology (Shanghai) Co., Ltd.**
  **Shanghai 201210 (CN)**
- **The First Affiliated Hospital, Zhejiang University School of Medicine**
  **Hangzhou, Zhejiang 310003 (CN)**

(72) Inventors:
- **YAO, Hangping**
  **Hangzhou, Zhejiang 310003 (CN)**
- **HUANG, Fude**
  **Shanghai 201210 (CN)**
- **LI, Lanjuan**
  **Shanghai 201210 (CN)**
- **WANG, Wenan**
  **Shanghai 201210 (CN)**
- **CAO, Luxiang**
  **Shanghai 201210 (CN)**
- **JIAO, Changping**
  **Shanghai 201210 (CN)**
- **WU, Nanping**
  **Shanghai 201210 (CN)**
- **LU, Xiangyun**
  **Shanghai 201210 (CN)**

(74) Representative: **Engelhard, Markus**
**Boehmert & Boehmert**
**Anwaltspartnerschaft mbB**
**Pettenkoferstrasse 22**
**80336 München (DE)**

(54) **ANTI-CORONAVIRUS EFFECT AND APPLICATION OF PI4K INHIBITOR**

(57) The use of phenylarsenic oxide and a derivative thereof in the prevention or treatment of coronavirus diseases, meanwhile also provided is the use of a PI4KIIIα specific inhibitor in the prevention or treatment of coronavirus diseases.



Processed by Luminess, 75001 PARIS (FR)

(Cont. next page)

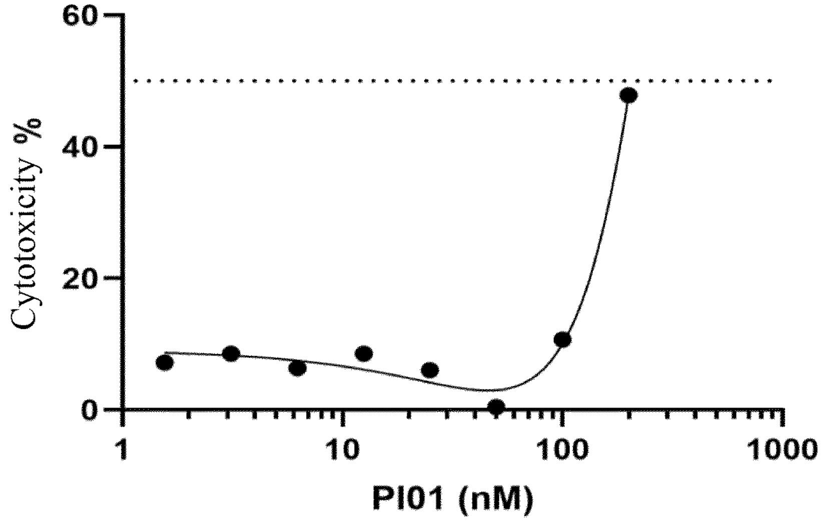


*FIG. 1A*

**Description**

Technical Field

The present invention relates to use of a PI4KIIIα specific inhibitor in the preparation of a drug for preventing or treating coronavirus diseases.

Background Art

Coronavirus has been of great interest because of its high infectivity. For example, case fatality ratios of two highly pathogenic coronaviruses severe, acute respiratory syndrome coronavirus (SARS-CoV) and middle east respiratory syndrome coronavirus (MERS-CoV), are as high as 10% and 36%, respectively; human coronaviruses (HCoV-229E, HCoV-OC43, HCoV-NL63, HCoV-HKU1 and HCoV-HKU8) cause 15% to 30% of human upper respiratory tract infection each year, and have a higher incidence of causing more severe diseases among newborns, the elderly and individuals with underlying diseases, therefore leading to a great attention to the coronaviruses.

It is generally believed that coronavirus enters into a host cell via a pH-dependent pathway, that is, when the virus is absorbed on a cell surface, the cell membrane is invaginated, the virus is internalized, and the viral RNA is released into a cytoplasm. Thereafter, the viral RNA-dependent RNA polymerase (RdRp) is first synthesized, and the polymerase recognizes a positive-strand RNA of the coronavirus genome and synthesizes a negative-strand RNA with the positive-strand RNA as a template, and then synthesizes subgenomic small-fragment positive-strand RNA and a positive-strand genomic RNA with the negative-strand RNA as a template. In the cytoplasm, a precursor protein is translated by the ribosome with the subgenomic small-fragment positive-strand RNA as a template. Thereafter, protein N binds to the newly synthesized genomic RNA, reaches the endoplasmic reticulum in the involvement of protein M, integrates with protein S and is released from the endoplasmic reticulum membrane. Meanwhile, the protease cleaves the precursor protein of the progeny virus into mature protein. Thereafter, the progeny virus is transferred from Golgi apparatus to the cell membrane and released outside the cell (Masters et al., The molecular biology of coronaviruses. Adv Virus Res. 2006. Vol 66, p. 193-292) for a new round of cell infection, intracellular replication and cell damage, thereby affecting normal functions of the body.

Recently, the novel coronavirus (SARS-CoV-2), also referred to as the new coronavirus, has attracted the attention of various countries due to very high infectivity of the novel coronavirus pneumonia (NCP) with basic reproduction number (RO) expected to be between 2 and 6 caused by the novel coronavirus. Like other coronaviruses, SARS-CoV-2 is a virus with an envelope. The genetic material of SARS-CoV-2 is a positive-strand single-stranded RNA virus, and its gene sequence is of the same lineage as SARS virus and MERS virus.

It is currently speculated that SARS-CoV-2, like other coronaviruses, stimulates the innate immune system of a patient, resulting in a massive release of cytokines in the body, and thus leading to a cytokine storm and an acute inflammatory response. This can lead to more fragile systemic blood vessels, leading to acute respiratory distress and multiple organ failure (Huang et al., Clinical features of patients infected with 2019 novel coronavirus in Wuhan, China. Lancet. 2020: p1-10; Kikkert et al., Innate Immune Evasion by Human Respiratory RNA Viruses. Journal of Innate Immunity. 2020, 12 (1)). The latest evidence shows that SARS-CoV-2 is present in the cerebrospinal fluid of patients with novel coronavirus pneumonia, and is consistent with symptoms such as neck rigidity, sudden disturbance of consciousness and coma in patients, which indicates that the virus can also attack the nervous system in addition to multiple organs such as lung, heart, blood and liver.

Similar to SARS-CoV, SARS-CoV-2 protein S can recognize and bind to the host surface receptor, angiotensin-converting enzyme 2 (ACE2), allowing the virus to absorb on the cell surface. ACE2 is believed to bind more strongly to SARS-CoV-2 than to SARS-CoV (Xu et al., Evolution of the novel coronavirus from the ongoing Wuhan outbreak and modeling of its spike protein for risk of human transmission. SCIENCE CHINA Life Sciences. 2020, Vol 63, p.457-460). Currently, no drug with a significant therapeutic effect on SARS-CoV-2 infection has been reported.

Phenylarsine oxide (PAO) is a known biological inhibitor,

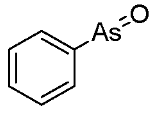

(I)

the arsenic atom in the molecule has high affinity for the sulfur atom in the mercapto group in a biomolecule. At present, it has not been reported that phenylarsine oxide and a derivative thereof may be used to treat coronavirus (including the novel coronavirus) diseases.

Summary of the Invention

In one aspect, the present invention provides use of a PI4KIIIα specific inhibitor in the preparation of a drug for preventing or treating coronavirus-like diseases.

In some embodiments, the PI4KIIIα specific inhibitor is an antibody, a small molecule compound, an RNAi molecule or an antisense nucleic acid.

In some embodiments, the antibody is a monoclonal antibody or a polyclonal antibody.

In some embodiments, the antibody is a chimeric antibody, a humanized antibody or a fully human antibody.

In some embodiments, the RNAi molecule is a small interference RNA (siRNA), a short hairpin RNA (shRNA) or a microRNA (miRNA).

In some embodiments, the RNAi molecule is 18-100 bases in length.

In some embodiments, the RNAi molecule is modified to enhance its stability.

In some embodiments, the PI4KIIIα specific inhibitor is a small molecule compound.

In some embodiments, the small molecule compound is phenylarsine oxide or a derivative thereof, G1 and an analog thereof, A1 and an analog thereof, or Simeprevir and an analog thereof.

In some embodiments, the phenylarsine oxide and the derivative thereof have a structure as represented by formula (I) or a pharmaceutically acceptable salt thereof,

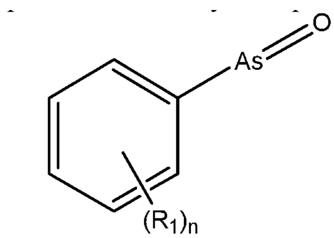

Formula (I)

wherein each $R_1$ is independently selected from (a) H, halogen, nitro, cyano, hydroxy, amino, carbamoyl, $C_{1-6}$ alkyl, $C_{2-6}$ alkynyl, $C_{2-6}$ alkenyl, $C_{1-6}$ alkoxy, $C_{1-6}$ haloalkyl, $C_{1-6}$ alkylene-$NH_2$, $C_{1-6}$ alkylene-NH-C(O)H, -As(O), -N=NH, N-($C_{1-6}$ alkyl)amino, *N*,*N*-($C_{1-6}$ alkyl)$_2$ amino, -NH-C(O)H, -NH-S(O)$_2$H, -C(O)OH, -OC(O)H, -SH, -S(O)$_2$H, -S(O)$_2$-$NH_2$ or heterocyclyl, and optionally substituted with $R_2$ or $R_3$, wherein the $R_2$ and the $R_3$ are each independently selected from amino, $C_{1-6}$ alkyl, $C_{1-6}$ alkoxy, $C_{1-6}$ haloalkyl, *N*-($C_{1-6}$ alkyl) amino, *N*-(6-12 membered aryl)amino, *N*,*N*-($C_{1-6}$ alkyl)$_2$ amino, $C_{3-6}$ cycloalkyl, 6-12 membered aryl or 3-12 membered heterocyclyl, and optionally substituted with one or more halogen, nitro, cyano, hydroxy, amino, carbamoyl, -NH-C(O)-$R_5$, -C(O)O$R_4$, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, $R_4$ is $C_{1-6}$ alkyl, and optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, and $R_5$ is selected from H, $C_{1-6}$ alkyl, $C_{2-6}$ alkynyl, $C_{2-6}$ alkenyl, $C_{1-6}$ alkoxy or $C_{1-6}$ haloalkyl, and/or

(b) $R_1$ on two adjacent carbon atoms forms 5-12 membered cycloalkyl, aryl or heterocyclyl, and is optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-,

wherein n is an integer from 0 to 5.

In some embodiments, n is an integer from 0 to 2, and each of the $R_1$ is independently selected from H, halogen, nitro, cyano, hydroxy, amino, carbamoyl, $C_{1-6}$ alkyl, $C_{1-6}$ alkoxy, $C_{1-6}$ haloalkyl, -As(O), *N*-($C_{1-6}$ alkyl)amino, *N*,*N*-($C_{1-6}$ alkyl)$_2$ amino, -NH-C(O)H or -NH-S(O)$_2$H, and optionally substituted with the $R_2$ or the $R_3$.

In some embodiments, n is an integer from 0 to 2, and each of the $R_1$ is independently selected from H, halogen, nitro, cyano, hydroxy, amino, $C_{1-6}$ alkyl, $C_{1-6}$ alkoxy, $C_{1-6}$ haloalkyl, -As(O), -NH-C(O)H or -NH-S(O)$_2$H, and optionally substituted with the $R_2$ or the $R_3$.

In some embodiments, n is 1 or 2, each of the $R_1$ is independently selected from H, halogen, amino, $C_{1-6}$ alkoxy, $C_{1-6}$ haloalkyl, -NH-C(O)$R_2$ or -NH-S(O)$_2R_3$, wherein the $R_2$ is $C_{1-6}$ alkyl and optionally substituted with one 6-12 membered aryl, and the $R_3$ is 6-12 membered aryl and optionally substituted with one halogen, $C_{1-6}$ alkoxy or $C_{1-6}$ haloalkyl.

In some embodiments, the $R_1$ is located at ortho- and/or para-position of -As(O).

In some embodiments, n is 0.

In some embodiments, the small molecule compound is selected from the group consisting of the following

compounds:

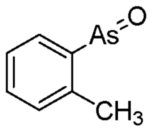, 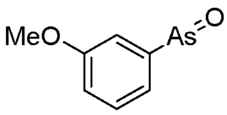, 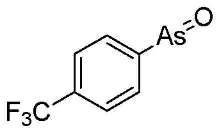, 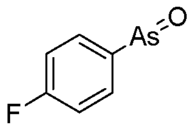, 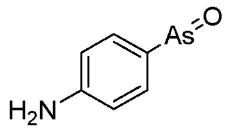,

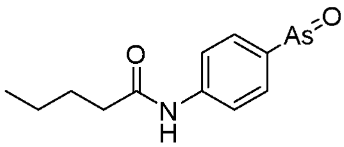, 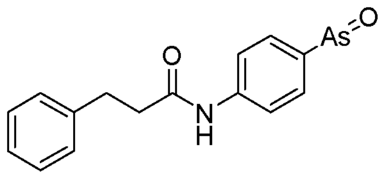, 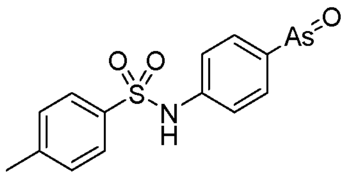,

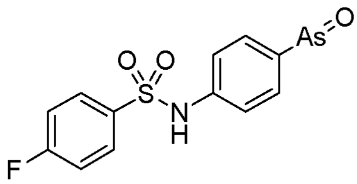, 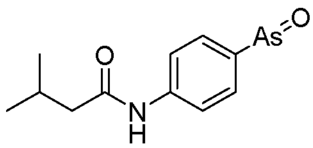, 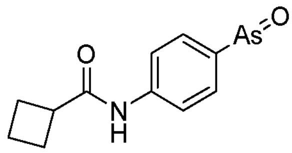,

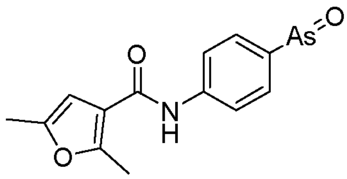, 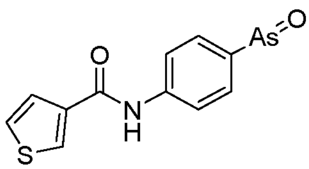, 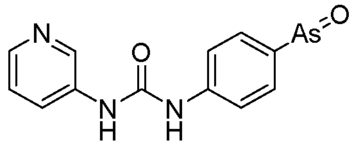,

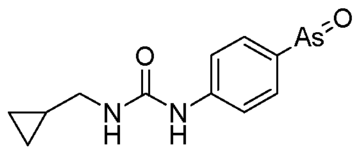, 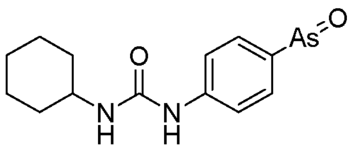, 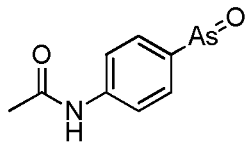,

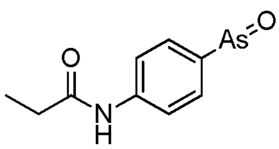, 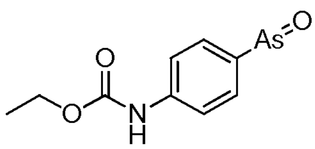, 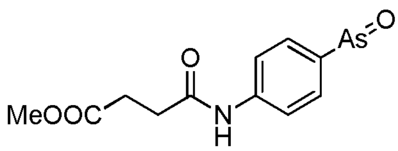,

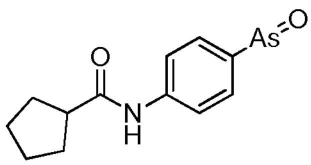, 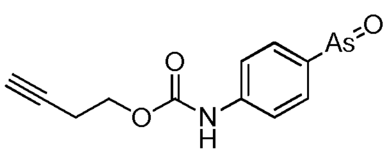, 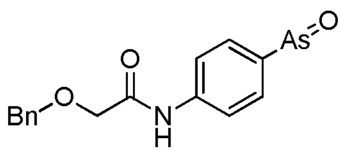,

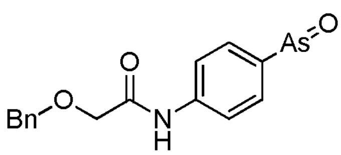, 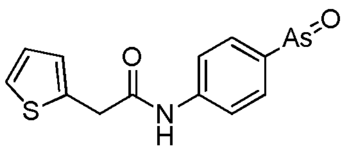, 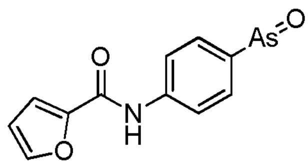,

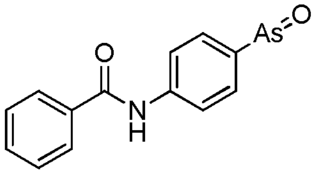, 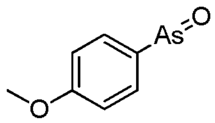, 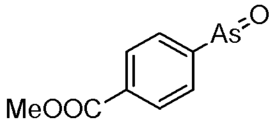, 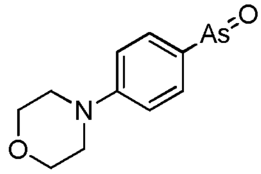,

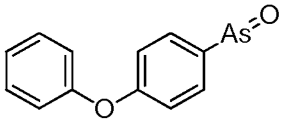, 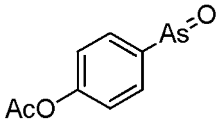, 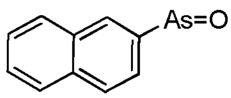, 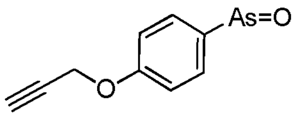,

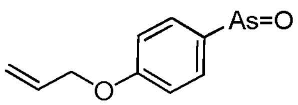, 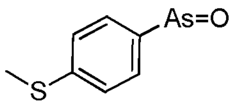, 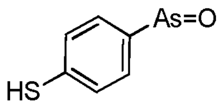, 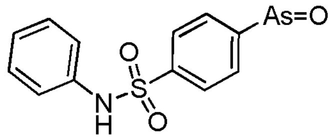,

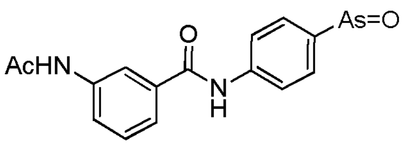, 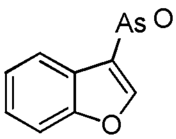, 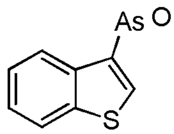, 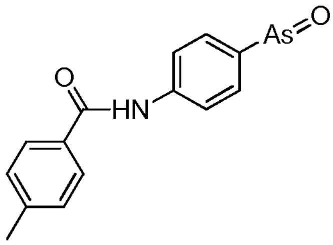,

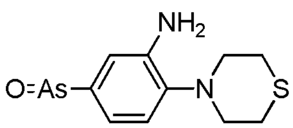, 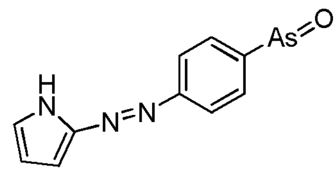, 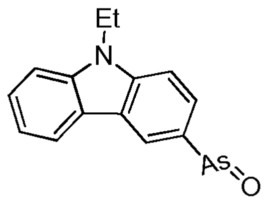,

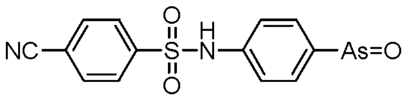, 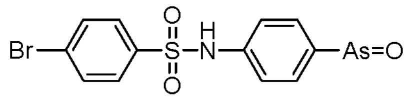,

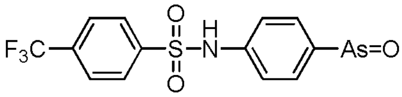, 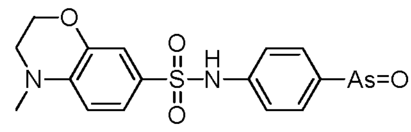, 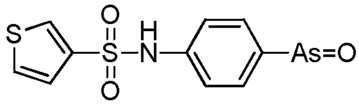,

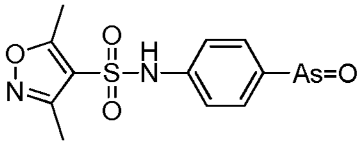, 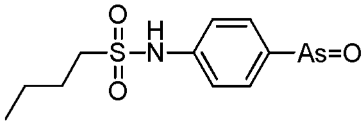, 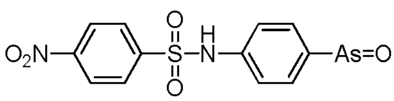.

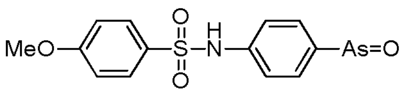 , 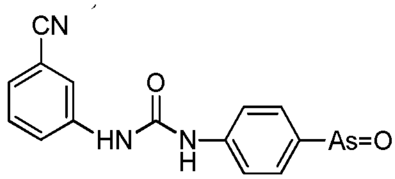 ,

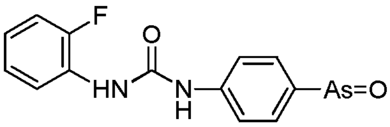 , 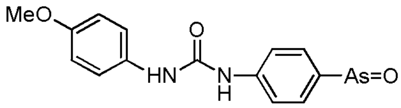 , 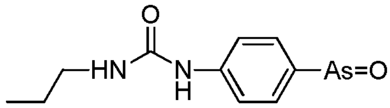 ,

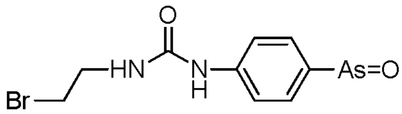 , 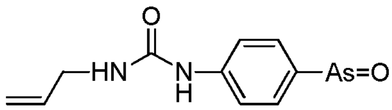 ,

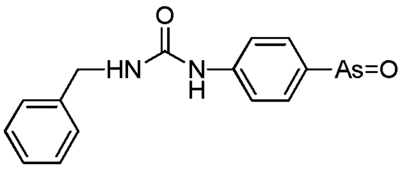 , 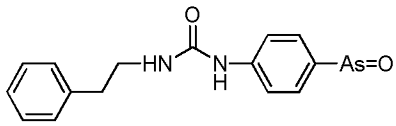 , 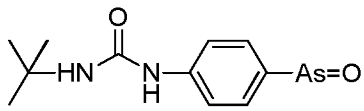 ,

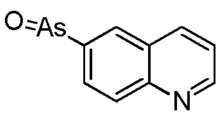 , 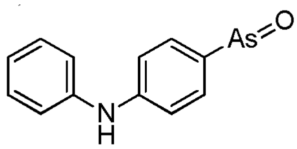 , 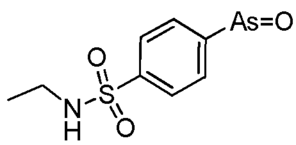 ,

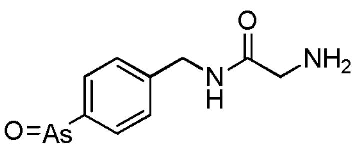 , 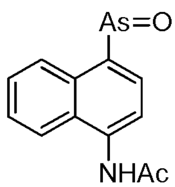 , 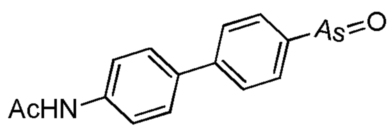 ,

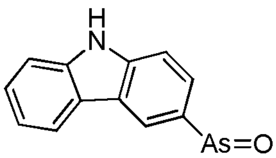 , 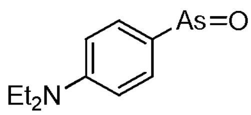 , 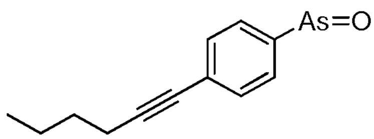 ,

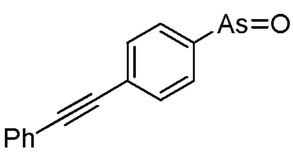 , 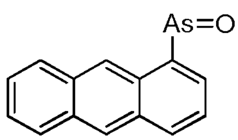 , 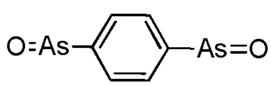 , 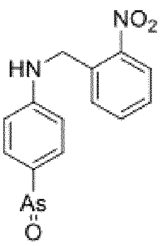 ,

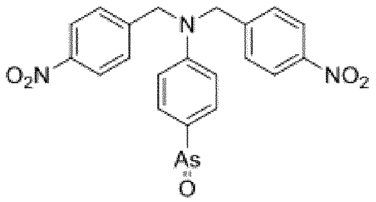,
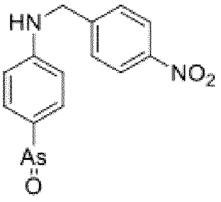,
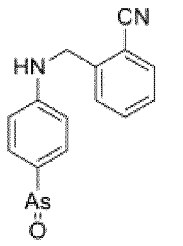,
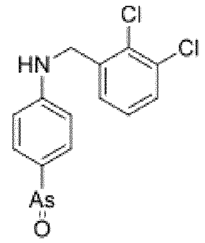,
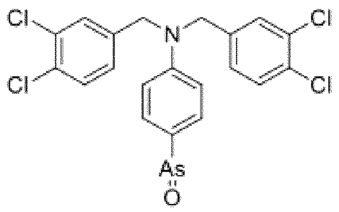
and
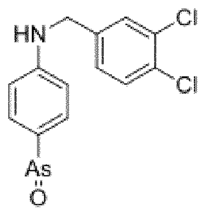.

In some embodiments, the subject is a human or a mammal.

In some embodiments, the coronavirus is a novel coronavirus.

In some embodiments, the coronavirus is chicken infectious bronchitis virus, porcine epidemic diarrhea virus, porcine transmissible gastroenteritis virus, porcine hemagglutinating encephalomyelitis virus, porcine δ-coronavirus, canine respiratory coronavirus, mouse hepatitis virus, feline coronavirus, human coronavirus, severe acute respiratory syndrome virus and middle east respiratory syndrome virus.

In some embodiments, administering a second agent to a subject in need thereof is further comprised.

In some embodiments, the second agent is an agent for treating coronavirus diseases.

In some embodiments, the PI4KIIIα specific inhibitor is administered prior to, subsequent to or concurrently with the second agent.

In another aspect, the present invention provides a method for screening a drug for preventing or treating coronavirus diseases, comprising contacting a drug candidate with a PI4KIIIα protein or nucleic acid or PI4KIIIα, and detecting whether the drug candidate is capable of inhibiting the formation or activity of PI4KIIIα.

In yet another aspect, the present invention provides use of the compound of formula (I) in the preparation of a drug for preventing or treating coronavirus diseases:

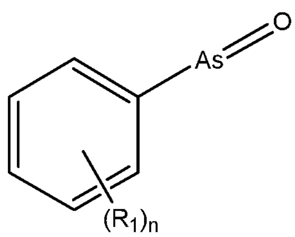

Formula (I)

wherein each $R_1$ is independently selected from (a) H, halogen, nitro, cyano, hydroxy, amino, carbamoyl, $C_{1-6}$ alkyl, $C_{2-6}$ alkynyl, $C_{2-6}$ alkenyl, $C_{1-6}$ alkoxy, $C_{1-6}$ haloalkyl, $C_{1-6}$ alkylene-$NH_2$, $C_{1-6}$ alkylene-NH-C(O)H, -As(O), -N=NH, N-($C_{1-6}$ alkyl)amino, *N,N*-($C_{1-6}$ alkyl)$_2$ amino, -NH-C(O)H, -NH-S(O)$_2$H, -C(O)OH, -OC(O)H, -SH, -S(O)$_2$H, -S(O)$_2$-$NH_2$ or heterocyclyl, and optionally substituted with $R_2$ or $R_3$, wherein the $R_2$ and the $R_3$ are each independently selected from amino, $C_{1-6}$ alkyl, $C_{1-6}$ alkoxy, $C_{1-6}$ haloalkyl, *N*-($C_{1-6}$ alkyl) amino, *N*-(6-12 membered aryl)amino, *N,N*-($C_{1-6}$ alkyl)$_2$ amino, $C_{3-6}$ cycloalkyl, 6-12 membered aryl or 3-12 membered heterocyclyl, and optionally substituted with one or more halogen, nitro, cyano, hydroxy, amino, carbamoyl, -NH-C(O)-$R_5$, -C(O)O$R_4$, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, $R_4$ is $C_{1-6}$ alkyl, and optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, and $R_5$ is selected from H, $C_{1-6}$ alkyl, $C_{2-6}$ alkynyl, $C_{2-6}$ alkenyl, $C_{1-6}$ alkoxy or $C_{1-6}$ haloalkyl, and/or

(b) $R_1$ on two adjacent carbon atoms forms 5-12 membered cycloalkyl, aryl or heterocyclyl, and is optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, wherein n is an integer from 0 to 5.

In some embodiments, the compound is phenylarsine oxide.

Brief Description of the Drawings

FIG. 1 shows the toxic effect of phenylarsine oxide at different concentrations on Vero cells. FIG. 1A shows the toxic effect of small molecule drug PI01 at a concentration below 200 nM on Vero cells, FIG. 1B shows the toxic effect of small molecule drug PI01 at a concentration of 200 nM on Vero cells, FIG. 1C shows the toxic effects of Remdesivir at different concentrations on Vero cells, and FIG. ID shows the toxic effects of Chloroquine phosphate (Chloroquine) at different concentrations on Vero cells.

FIG. 2 shows the *in vitro* viral inhibitory effects of phenylarsine oxide at different concentrations. FIG. 2A shows the effects of drug PI01 at different concentrations on inhibition of SARS-CoV-2 in Vero cells, FIG. 2B shows the effects of drug PI01 at different concentrations on inhibition of SARS-CoV-2 in Vero cells, FIG. 2C shows the effects of Remdesivir at different concentrations on inhibition of SARS-CoV-2 in Vero cells, and FIG. 2D shows the effects of Chloroquine phosphate at different concentrations on inhibition of SARS-CoV-2 in Vero cells.

FIG. 3 shows the activity of different agents against human coronavirus HCoV-229E in MRC5 cells. FIG. 3A shows the results for the first round, and FIG. 3B shows the results for the second round.

Detailed Description of The Invention

Hereinafter, the present invention will be described in detail according to the embodiments and in conjunction with the accompanying drawings. The above aspects of the present invention and other aspects of the present invention will be apparent from the following detailed description. The scope of the present invention is not limited to the following embodiments.

PI4KIIIα specific inhibitor

The term "PI4KIIIα specific inhibitor" as used herein refers to various substances capable of specifically reducing, decreasing or eliminating the transcription or translation of PI4KIIIα gene, and/or the activity of PI4KIIIα protein. In some embodiments, the PI4KIIIα specific inhibitor is capable of reducing the activity of PI4KIIIα by at least 5%, 10%, 20%, 40%, 50%, 80%, 90%, 95% or more. As used herein, "activity" when used in conjunction with an increase or decrease refers to a detected functional activity, which may be present in varying amounts, or in a constant amount but with a varying functional activity.

As used herein, the activity of PI4KIIIα refers to an activity of PI4KIIIα protein to phosphorylate phosphatidylinositol (PI) at a specific position (for example, to convert to phosphatidylinositol 4-phosphate (PI4P)). The binding constant of the PI4KIIIα specific inhibitor to PI4KIIIα protein is at least 2 times of the binding constant of the PI4KIIIα specific inhibitor to other non-specific binding proteins. In some embodiments, the PI4KIIIα specific inhibitor is capable of preferably recognizing PI4KIIIα protein in a complex mixture, including a mixture with other PI4K subtype proteins.

In some embodiments, the PI4KIIIα specific inhibitor inhibits PI4KIII at least 1 time, 2 times, 4 times, 5 times, 10 times, 20 times, 30 times, 50 times, 100 times, 200 times, 500 times, or 10,000 times more than other subtypes of PI4K protein (e.g., PI4K protein, including PI4KIIα or PMKIIβ). For example, in some embodiments, the PI4KIIIα specific inhibitor does not substantially inhibit PI4KII (e.g., PI4KIIα or PMKIIβ), e.g., $IC_{50}$ of the PI4KIIIα specific inhibitor is greater than or equal to 10 μM, 20 μM, 30 μM, 40 μM, 50 μM, 60 μM, 80 μM, 100 μM, 150 μM, 200 μM or 500 μM.

In some embodiments, the PI4KIIIα specific inhibitor inhibits PI4KIIIα at least 1 time, 2 times, 4 times, 5 times, 10 times, 20 times, 30 times, 50 times, 100 times, 200 times, 500 times, or 10,000 times stronger than other subtypes of PI4KIII (e.g., PMKIIIβ).

In some embodiments, $IC_{50}$ of PI4KIIIα specific inhibitor against PI4KIIIα is less than or equal to 100 μM, 80 μM, 50 μM, 30 μM, 20 μM, 10 μM, 5 μM, 3 μM, 2 μM, 1 μM, 0.5 μM, 0.2 μM, 0.1 μM, 0.05 μM, 0.02 μM, 0.01 μM, 0.005 μM, 0.002 μM or 0.001 μM. In some embodiments, the PI4KIIIα specific inhibitor is an antibody, a small molecule compound, an RNAi molecule or an antisense nucleic acid.

In some embodiments, the PI4KIIIα specific inhibitor is an antibody.

The term "antibody" as used herein encompasses any immunoglobulin, monoclonal antibody, polyclonal antibody, multivalent antibody, bivalent antibody, monovalent antibody or antibody that binds a specific antigen. The term

"antibody" herein is intended to encompass conventional four-chain antibodies as well as less conventional antibodies without four chains (e.g., antibodies naturally devoid of light chains).

One conventional intact antibody is a heterotetramer comprising two heavy (H) chains and two light (L) chains. Heavy chains of mammals may be classified into α chain, δ chain, ε chain, γ chain and μ chain, and each heavy chain consists of a variable region (VH) and a first constant region, a second constant region and a third constant region (CH1, CH2 and CH3, respectively); and light chains of mammals may be classified into λ chain or κ chain, and each light chain consists of a variable region (VL) and a constant region. A conventional antibody is "Y"-shaped, with the neck of the "Y"-shaped structure consisting of a second constant region and a third constant region of two heavy chains bound by a disulfide bond. Each arm of the "Y"-shaped structure comprises a variable region and a first constant region of one heavy chain, which bind to a variable region and a constant region of one light chain. The variable regions of the light and heavy chains determine antigen binding. The variable region of each chain contains three hypervariable regions, i.e., complementarity determining regions (CDRs) (the CDRs for the light chain include LCDR1, LCDR2 and LCDR3, and the CDRs for the heavy chain include HCDR1, HCDR2 and HCDR3). The three CDRs are interposed between flanking continuous portions known as framework regions (FRs), which are more highly conserved than the CDRs and form a scaffold to support the hypervariable loops. The constant regions of the heavy and light chains are not associated with antigen binding, but have multiple effector functions.

In some embodiments of the present invention, the antibody is a full-length antibody or an antigen-binding fragment.

The term "antigen-binding fragment" as used herein refers to an antibody fragment formed from the antibody fragment that contains an antibody portion with one or more CDRs but does not have an intact antibody structure. Examples of the antigen-binding fragment include, but are not limited to, an Fab fragment, an Fab' fragment, an F(ab')2 fragment, an Fv fragment, a single chain antibody molecules (scFv), an scFv dimer, a camelized single domain antibody and a nanobody. The antigen-binding fragment may bind to the same antigen as the parent antibody.

The "Fab" fragment of an antibody refers to the antibody portion that contains one light chain (including a variable region and a constant region) and a variable region and a first constant region of one heavy chain bound by a disulfide bond.

The "Fab'" fragment refers to an Fab fragment containing part of the hinge region.

The "F(ab')2" fragment refers to a dimer of Fab'.

The "Fv" fragment of an antibody consists of a variable region of one light chain and a variable region of one heavy chain.

The "single chain antibody molecule" or "scFv" refers to an engineered antibody composed of a light chain variable region and a heavy chain variable region directly connected or connected by a peptide chain. For detailed descriptions, see, e.g., Huston JS et al., Proc Natl A cad Sci USA, 85:5879 (1988).

The "scFv dimer" refers to a polymer formed from two scFvs.

The "camelized single domain antibody" (also referred to as "heavy chain antibody" or "heavy-chain-only antibodies (HCAb)") refers to an antibody that contains two heavy chain variable regions but no light chain. Heavy chain antibodies are originally derived from Camelidae (camels, dromedaries and llamas). Camelized antibodies have all the functions for antigen binding despite the deletion of light chains.

The "nanobody" consists of one heavy chain variable region and two constant regions CH2 and CH3 from a heavy chain antibody.

In some embodiments, the antibody is a monoclonal antibody or a polyclonal antibody.

In some embodiments, the antibody is a murine antibody, a rabbit antibody, a chimeric antibody, a humanized antibody or a fully human antibody.

The term "fully human" as used herein, when applied to an antibody or antigen-binding fragment, means that amino acid sequences of the antibody or antigen-binding fragment correspond to the amino acid sequence of an antibody produced by human or human immune cells or derived from a non-human source, e.g., a transgenic non-human animal utilizing a human antibody library, or other sequences encoding human antibodies.

The term "humanized" as used herein, when applied to an antibody or antigen-binding fragment, refers to an antibody or antigen-binding fragment that includes CDRs derived from a non-human animal, FR regions derived from a human, and constant regions derived from a human (when applicable). Because a humanized antibody or antigen-binding fragment has lower immunogenicity, it may be used as a therapeutic agent for human in certain embodiments. In certain embodiments, the non-human animals are mammals (e.g., mice, rats, rabbits, goats, sheep, guinea pigs or hamsters). In certain embodiments, the humanized antibody or antigen-binding fragment consists essentially entirely of human sequences, except for CDR sequences that are non-human.

The term "chimeric" as used herein, when applied to an antibody or antigen-binding fragment, refers to having a portion of the heavy and/or light chain derived from one species, with the rest of the heavy and/or light chain derived from an antibody or antigen-binding fragment from different species. In some embodiments, the chimeric antibody can include a constant region derived from a human and a variable region derived from a non-human animal (e.g., a mouse

or a rabbit).

In some embodiments, the antibody described herein is a monospecific antibody, a bispecific antibody or a multispecific antibody.

In some embodiments, the antibody described herein may be further labeled.

In some embodiments, the PI4KIIIα specific inhibitor is an RNAi molecule.

The term "RNAi molecule" as used herein refers to RNA or an analog thereof, which has sufficient sequence complementarity with target RNA to guide RNA interference. In some embodiments, DNA is also included that may be used to generate RNA. RNA interference (RNAi) refers to a sequence-specific or selective process by which a target molecule (e.g., a target gene, a protein or RNA) is down-regulated.

In some embodiments, the RNAi molecule is capable of reducing expression of PI4KIIIα, e.g., knocking down PI4KA gene.

In some embodiments, the RNAi molecule is 18-100 bases in length.

In some embodiments, the RNAi molecule is modified to enhance its stability.

In some embodiments, the RNAi molecule is a small interference RNA (siRNA), a short hairpin RNA (shRNA) or a microRNA (miRNA).

The term "small interference RNA (siRNA)" as used herein refers to an RNA molecule, preferably a double-strand molecule, having a length of about 10 to 50 nucleotides, preferably a length of about 15 to 25 nucleotides, more preferably a length of about 17, 18, 19, 20, 21, 22, 23, 24 or 25 nucleotides, and the strand optionally has an overhanging terminus comprising, e.g., 1, 2 or 3 overhanging nucleotides (or nucleotide analogs). siRNA is capable of guiding or mediating degradation of RNA.

The term "short hairpin RNA (shRNA)" as used herein refers to an RNA molecule having a stem-loop structure that includes a first region and a second region of complementary sequences, complementarity degree and region direction are sufficient for base pairing to occur between the regions, the first and second regions are linked by a loop region, and the loop results from a lack of base pairing between nucleotides (or nucleotide analogs) in the loop region.

The term "microRNA or miRNA" as used herein is a short, naturally occurring, non-coding and single-stranded RNA molecule of about 16-26 nucleotides (nt) in length (e.g., about 16-29 nt, 19-22 nt, 20-25 nt or 21-23 nt), which is typically involved in regulating gene expression *in vivo.* In eukaryotic cells, the miRNA gene is transcribed by DNA transcriptase II into a "primary miRNA" (pri-miRNA) that is rapidly processed by ribonuclease III (Drosha) into a "precursor" miRNA (pre-miRNA), and the pre-miRNA is transported from cell nucleus into cytoplasm, and then recognized and cleaved into mature miRNA by another ribonuclease III (Dicer). Mature miRNA molecules are partially complementary to one or more mRNAs and regulate the expression of proteins. The sequences of known miRNAs may be obtained from published databases, such as the miRBase database (www.mirbase.org), where information is provided including miRNA sequence information, functional annotations, predicted gene targets and the like. In the present invention, miRNA further includes RNA molecules expressed in cells by synthetic plasmids, having a structure and function similar to natural miRNA, and capable of targeting the corresponding mRNA like natural miRNA and preventing its translation into protein.

In some embodiments, the PI4KIIIα specific inhibitor is an antisense nucleic acid.

The term "antisense nucleic acid" as used herein includes nucleotides that are fully complementary to a target sequence as well as those nucleotides that have one or more nucleotide mismatches, so long as the antisense nucleic acid is capable of specifically hybridizing to the target sequence. For example, the antisense nucleic acid herein includes a polynucleotide having at least 70% or more, preferably 80% or more, more preferably 90% or more, and even more preferably 95% or more homology over a length of at least 15 consecutive nucleotides. Due to the formation of a hybrid, the transcription of the target gene and/or translation of the target mRNA is reduced or blocked.

In some embodiments, the PI4KIIIα inhibitor is a small molecule compound.

The term "small molecule compound" as used herein refers to an organic compound having a molecular weight of less than 3000, 2500, 2000, 1500, 1000 or 500 Daltons, which may be natural or chemically synthesized.

The small molecule compound has a structure as represented by formula (I) or a pharmaceutically acceptable salt thereof,

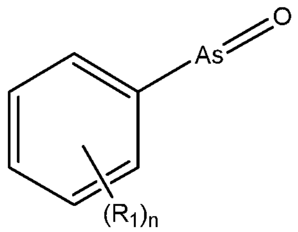

Formula (I)

wherein each $R_1$ is independently selected from (a) H, halogen, nitro, cyano, hydroxy, amino, carbamoyl, $C_{1-6}$ alkyl, $C_{2-6}$ alkynyl, $C_{2-6}$ alkenyl, $C_{1-6}$ alkoxy, $C_{1-6}$ haloalkyl, $C_{1-6}$ alkylene-$NH_2$, $C_{1-6}$ alkylene-NH-C(O)H, -As(O), -N=NH, N-($C_{1-6}$ alkyl)amino, *N*,*N*-($C_{1-6}$ alkyl)$_2$ amino, -NH-C(O)H, -NH-S(O)$_2$H, -C(O)OH, -OC(O)H, -SH, -S(O)$_2$H, -S(O)$_2$-NH$_2$ or heterocyclyl, and optionally substituted with $R_2$ or $R_3$, wherein the $R_2$ and the $R_3$ are each independently selected from amino, $C_{1-6}$ alkyl, $C_{1-6}$ alkoxy, $C_{1-6}$ haloalkyl, *N*-($C_{1-6}$ alkyl) amino, *N*-(6-12 membered aryl)amino, *N*,*N*-($C_{1-6}$ alkyl)$_2$ amino, $C_{3-6}$ cycloalkyl, 6-12 membered aryl or 3-12 membered heterocyclyl, and optionally substituted with one or more halogen, nitro, cyano, hydroxy, amino, carbamoyl, -NH-C(O)-$R_5$, -C(O)O$R_4$, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, $R_4$ is $C_{1-6}$ alkyl, and optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, and $R_5$ is selected from H, $C_{1-6}$ alkyl, $C_{2-6}$ alkynyl, $C_{2-6}$ alkenyl, $C_{1-6}$ alkoxy or $C_{1-6}$ haloalkyl, and/or

(b) $R_1$ on two adjacent carbon atoms forms 5-12 membered cycloalkyl, aryl or heterocyclyl, and is optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-,

wherein n is an integer from 0 to 5.

In some embodiments, the n is 0, 1, 2 or 3. In some embodiments, the n is 0, 1 or 2. In some embodiments, the n is 0 or 1.

In some embodiments, the n is an integer from 0 to 2, and each of the $R_1$ is independently selected from H, halogen, nitro, cyano, hydroxy, amino, carbamoyl, $C_{1-6}$ alkyl, $C_{1-6}$ alkoxy, $C_{1-6}$ haloalkyl, -As(O), *N*-($C_{1-6}$ alkyl)amino, *N*,*N*-($C_{1-6}$ alkyl)$_2$ amino, -NH-C(O)H or -NH-S(O)$_2$H, and optionally substituted with the $R_2$ or the $R_3$.

In some embodiments, the n is an integer from 0 to 2, and each of the $R_1$ is independently selected from H, halogen, nitro, cyano, hydroxy, amino, $C_{1-6}$ alkyl, $C_{1-6}$ alkoxy, $C_{1-6}$ haloalkyl, -As(O), -NH-C(O)H or -NH-S(O)$_2$H, and optionally substituted with the $R_2$ or the $R_3$.

In some embodiments, the n is 1 or 2, each of the $R_1$ is independently selected from H, halogen, amino, $C_{1-6}$ alkoxy, $C_{1-6}$ haloalkyl, -NH-C(O)$R_2$ or -NH-S(O)$_2R_3$, wherein the $R_2$ is $C_{1-6}$ alkyl and optionally substituted with one 6-12 membered aryl, and the $R_3$ is 6-12 membered aryl and optionally substituted with one halogen, $C_{1-6}$ alkoxy or $C_{1-6}$ haloalkyl.

In some embodiments, the $R_1$ is located at ortho and/or para-position of -As(O).

In some embodiments, the $R_1$ is H.

The term "substituted" as used herein, when referring to a chemical group, means that one or more hydrogen atoms of the chemical group are removed and substituted with a substituent.

The term "substituent" as used herein has general meaning known in the art and refers to a chemical moiety covalently attached to, or fused, where appropriate, to a parent group.

The term "$C_n$-$C_m$" as used herein represents a range of numbers of carbon atoms, where n and m are integers and the range of numbers of carbon atoms includes the endpoints (i.e., n and m) and each integer point therebetween. For example, $C_1$-$C_6$ represents a range of 1 to 6 carbon atoms, including 1 carbon atom, 2 carbon atoms, 3 carbon atoms, 4 carbon atoms, 5 carbon atoms and 6 carbon atoms.

The term "alkyl" as used herein, whether used as a part of another term or used alone, refers to a saturated hydrocarbon group, which may be linear or branched. The term "$C_n$-$C_m$ alkyl" refers to alkyl having n to m carbon atoms. In certain embodiments, the alkyl group contains 1 to 12, 1 to 8, 1 to 6, 1 to 4, 1 to 3, or 1 to 2 carbon atoms. Examples of the alkyl group include, but are not limited to, chemical groups such as methyl, ethyl, *n*-propyl, isopropyl, *n*-butyl, tert-butyl, isobutyl, sec-butyl, 2-methyl-1-butyl, *n*-pentyl, 3-pentyl, *n*-hexyl, 1,2,2-trimethylpropyl.

The term "alkenyl" as used herein, whether used as a part of another term or used alone, refers to an unsaturated hydrocarbyl group, which may be a linear or branched group having at least one carbon-carbon double bond. In certain embodiments, the alkenyl group contains 2 to 12, 2 to 10, 2 to 8, 2 to 6, 2 to 5, 2 to 4, or 2 to 3 carbon atoms. In certain

embodiments, the alkenyl group can also have 1 to 6, 1 to 5, 1 to 4, 1 to 3, 1 to 2, or 1 carbon-carbon double bond. Examples of the alkenyl group include, but are not limited to, chemical groups such as ethenyl, *n*-propenyl, isopropenyl, *n*-butenyl, *sec*-butenyl.

The term "alkynyl" as used herein, whether used as a part of another term or used alone, refers to an unsaturated alkynyl group, either linear or branched, having at least one carbon-carbon triple bond. In certain embodiments, the alkynyl group contains 2 to 12, 2 to 10, 2 to 8, 2 to 6, 2 to 5, 2 to 4, or 2 to 3 carbon atoms. In certain embodiments, the alkynyl group can also have 1 to 6, 1 to 5, 1 to 4, 1 to 3, 1 to 2, or 1 carbon-carbon triple bond. Examples of the alkynyl group include, but are not limited to, chemical groups such as ethynyl, propynyl and butynyl.

The term "cycloalkyl" as used herein refers to cyclic alkyl consisting of at least 3 atoms. The term "n-m membered cycloalkyl" refers to cycloalkyl having n to m members forming a ring. Furthermore, the ring may also have one or more double bonds, but does not have a fully conjugated system. In certain embodiments, the cycloalkyl has 3 to 8, 3 to 6, or 4 to 6 carbon atoms forming a ring. Examples of the cycloalkyl include, but are not limited to, cyclopropane, cyclobutane, cyclopentyl and the like.

The term "heterocyclyl" as used herein, refers to a cyclic group in which at least one atom in the ring system is a heteroatom and the remaining ring atoms are carbon atoms. The term "n-m membered heterocyclyl" refers to heterocyclyl having n to m members forming a ring. The term "heterocyclyl" as used herein includes heteroaryl and heterocycloalkyl. In addition, the ring may also have one or more double bonds. In certain embodiments, the heterocyclyl is saturated heterocycloalkyl. Examples of the heteroatom include, but are not limited to, oxygen, sulfur, nitrogen, phosphorus and the like.

The term "heterocycloalkyl" as used herein refers to cycloalkyl in which at least one atom in the ring system is a heteroatom and the remaining ring atoms are carbon atoms. The term "n-m membered heterocycloalkyl" refers to heterocycloalkyl having n to m members forming a ring. Furthermore, the ring may also have one or more double bonds, but does not have a fully conjugated system. In certain embodiments, the heterocycloalkyl is saturated heterocycloalkyl. Examples of the heteroatom include, but are not limited to, oxygen, sulfur, nitrogen, phosphorus and the like. In certain embodiments, the heterocycloalkyl has 3 to 8, 3 to 6, or 4 to 6 carbon atoms forming a ring. Examples of the heterocycloalkyl include, but are not limited to, azetidine, aziridine, pyrrolidinyl, piperidinyl, piperazinyl, morpholinyl, thiomorpholine, homopiperazine and the like.

The term "aryl" as used herein, whether used as a part of another term or used alone, refers to a mono-or polycarbocyclic ring system group having alternating double and single bonds between carbon atoms forming a ring. The term "$C_n$-$C_m$ aryl" refers to aryl having n to m carbon atoms forming a ring. In certain embodiments, the aryl ring system has 6 to 12, 6 to 10, or 6 to 8 carbon atoms in one or more rings. In certain embodiments, the aryl ring system has 2 or more rings fused together. Examples of the aryl group include, but are not limited to, chemical groups such as phenyl, naphthyl, tetrahydronaphthyl, indanyl, indenyl and the like.

The term "heteroaryl" as used herein refers to an aryl group in which at least one ring atom in the aromatic ring is a heteroatom and the remaining ring atoms are carbon atoms. The term "n-m membered heteroaryl" refers to heteroaryl having n to m members forming a ring. Examples of the heteroatom include, but are not limited to, oxygen, sulfur, nitrogen, phosphorus and the like. In certain embodiments, the heteroaryl can have 5 to 10, 5 to 8, or 5 to 6 members forming a ring. In certain embodiments, the heteroaryl is 5- or 6-membered heteroaryl. Examples of the heteroaryl include, but are not limited to, furyl, thienyl, pyridyl, quinolyl, pyrrolyl, *N*-lower alkylpyrrolyl, pyridyl-*N*-oxide, pyrimidinyl, pyrazinyl, imidazolyl, indolyl and the like.

The term "alkoxy" as used herein, whether used as a part of another term or used alone, refers to a group as represented by formula "-O-alkyl". The term "$C_n$-$C_m$ alkoxy" means that the alkyl moiety of the alkoxy has n to m carbon atoms. In certain embodiments, the alkyl moiety has 1 to 6, 1 to 4, or 1 to 3 carbon atoms. Examples of the alkoxy group include, but are not limited to, chemical groups such as methoxy, ethoxy, propoxy (e.g., *n*-propoxy and isopropoxy), *t*-butoxy and the like.

The term "haloalkyl" as used herein, whether used as a part of another term or used alone, refers to a group as represented by formula "-alkyl-X", wherein X is a halogen, an atom selected from fluorine, chlorine, bromine and iodine. The term "$C_n$-$C_m$ haloalkyl" means that the alkyl moiety of the haloalkyl has n to m carbon atoms. In certain embodiments, the alkyl moiety has 1 to 6, 1 to 4, or 1 to 3 carbon atoms. Examples of the haloalkyl group include, but are not limited to, chemical groups such as halomethyl, haloethyl, halopropyl (e.g., *n*-halopropyl and iso-halopropyl), *t*-halobutyl and the like.

The term "n-membered" as used herein, wherein n is an integer, is commonly used with a ring system to describe the number of atoms forming a ring in the ring system. For example, piperidinyl is one example of a 6-membered heterocycloalkyl ring, pyrazolyl is one example of a 5-membered heteroaryl ring, pyridinyl is one example of a 6-membered heteroaryl ring and 1,2,3,4-tetrahydro-naphthalene is one example of 10-membered aryl. The term "n- to m-membered" as used herein is generally used with a ring system to describe the number of members forming a ring in the ring system, where n and m are integers and the range of members forming a ring includes the endpoints (i.e., n and m) and each integer point therebetween. For example, 3- to 8-membered means a range of 3 to 8 members forming a ring, including

3 members, 4 members, 5 members, 6 members, 7 members and 8 members.

The term "halogen" as used herein refers to an atom selected from fluorine, chlorine, bromine and iodine.

The term "cyano" as used herein refers to a group as represented by formula "-CN".

The term "hydroxy" as used herein refers to a group as represented by formula "-OH".

The term "nitro" as used herein refers to a group as represented by formula "$-NO_2$".

The term "amino" as used herein refers to a group as represented by formula "$-NH_2$".

The term "carbamoyl" as used herein refers to a group as represented by formula "$-HNCONH_2$".

The term "compound" as used herein is intended to include all stereoisomers (e.g., enantiomers and diastereomers), geometric isomers, tautomers and isotopes of the structures shown.

The compounds described herein may be asymmetric (e.g., having one or more stereocenters). Unless otherwise indicated, all stereoisomers, such as enantiomers and diastereomers, are intended to be included. Various geometric isomers including olefins, carbon-carbon double bonds and the like may also be present in the compounds described herein, and all such stable isomers have been contemplated herein. *Cis-* and *trans-*geometric isomers of the compounds are described herein and may be isolated as mixtures of isomers or as individual isomers.

The compounds herein also include tautomeric forms of the compounds. Tautomeric forms result from the exchange of a single bond with an adjacent double bond accompanied by the migration of a proton. Tautomeric forms include tautomers of protons in isomeric protonated states with the same chemical formula and total charge. Examples of the proton tautomers include keto-enol pairs, amide-imidic acid pairs, lactam-lactim pairs, enamine-imine pairs and cyclic forms, in which a proton can occupy two or more positions of a heterocyclic system, such as 1H- and 3*H*-imidazole, *1H-, 2H-* and 4*H*-1,2,4-triazole, 1H- and 2*H*-isoindole and 1H- and 2*H*-pyrazole. Tautomeric forms can be in equilibrium or sterically locked into one form by appropriate substitution.

In certain embodiments, the small molecule compound herein can be obtained by organic synthesis. The compounds herein, including salts, esters, hydrates, or solvates thereof, may be prepared using any of the well-known organic synthetic techniques and may be synthesized according to a variety of possible synthetic routes.

In some embodiments, the small molecule compound described herein is a compound having the following structural formula, including one or more of:

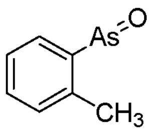 , 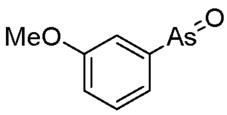 , 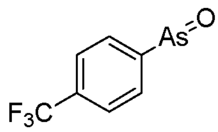 , 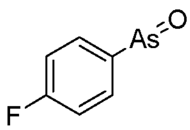 , 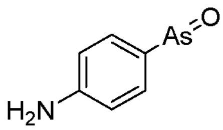 ,

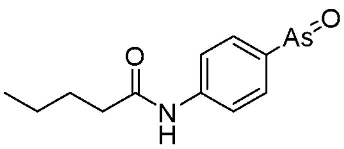 , 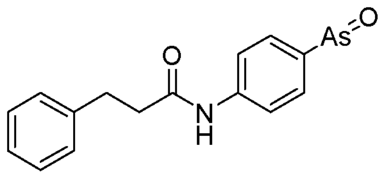 , 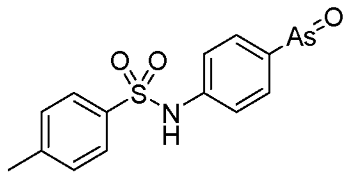 ,

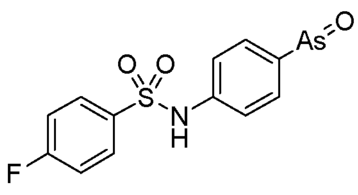 , 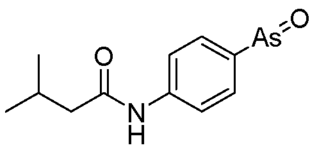 , 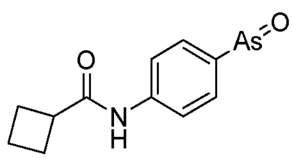 ,

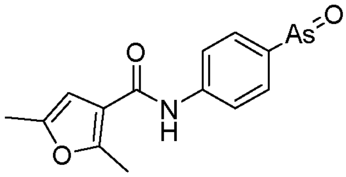 , 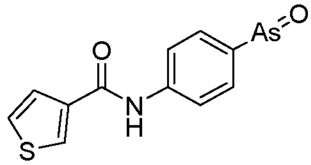 , 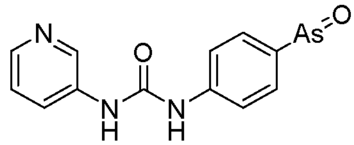 ,

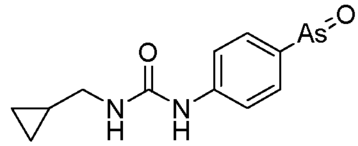,
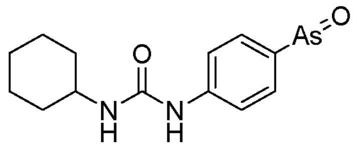,
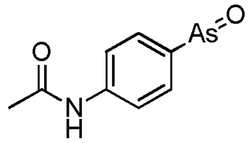,

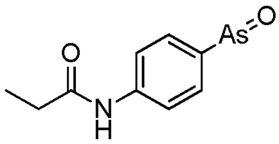,
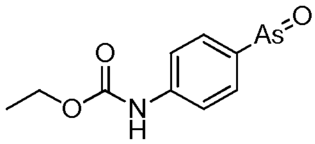,
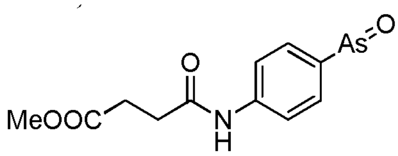,

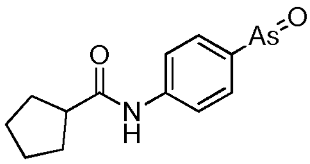,
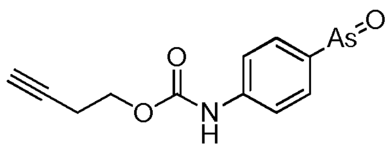,
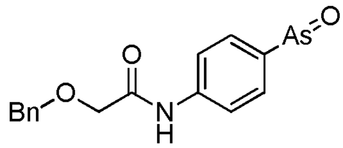,

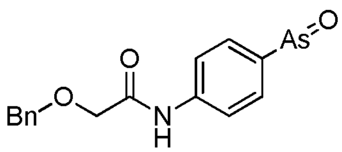,
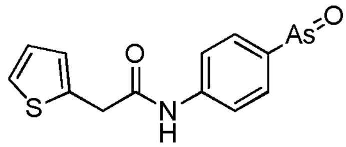,
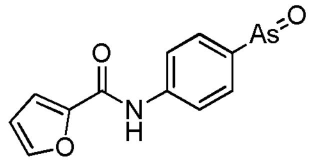,

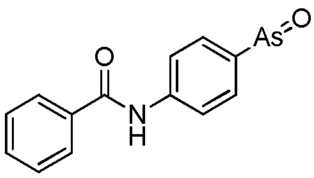,
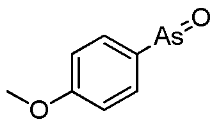,
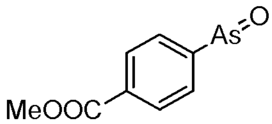,
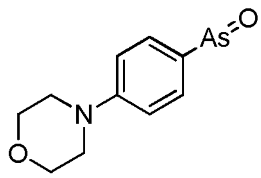,

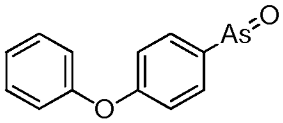,
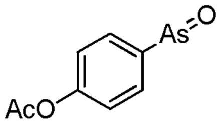,
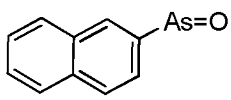,
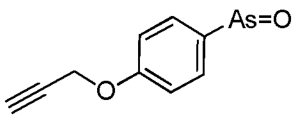,

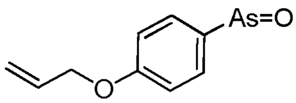,
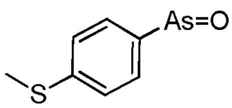,
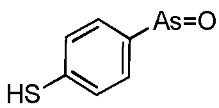,
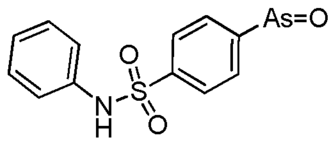,

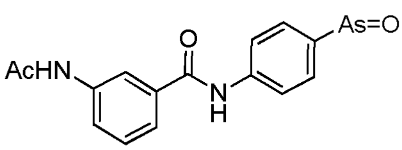,
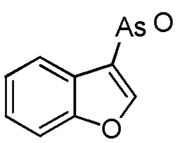,
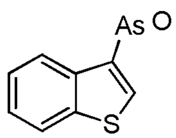,
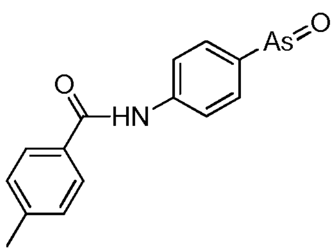,

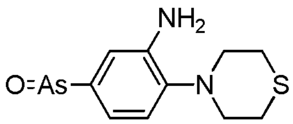,
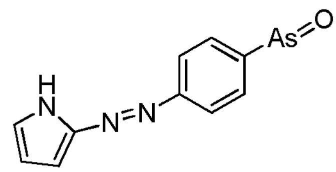,
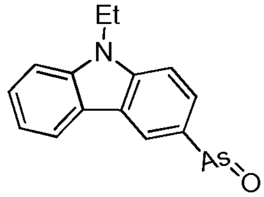,

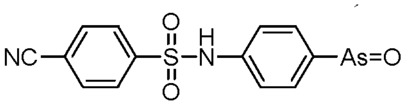,
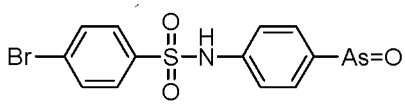

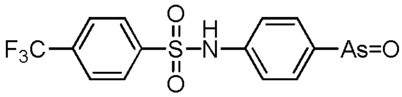,
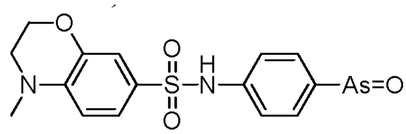,
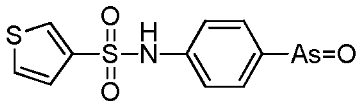,

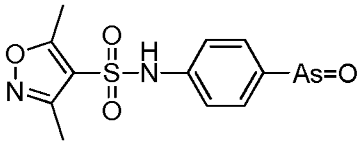,
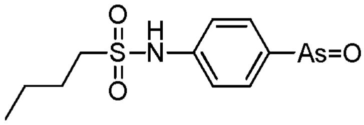,
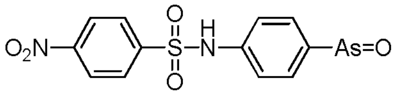,

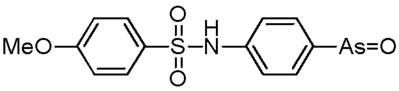,
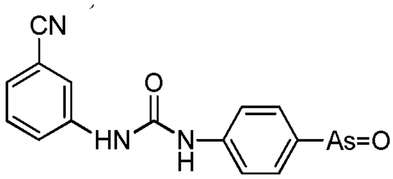

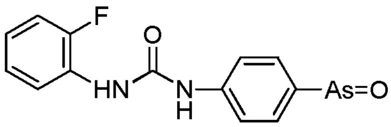,
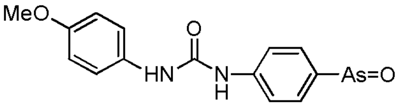,
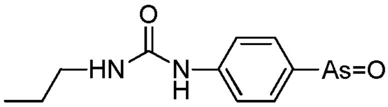,

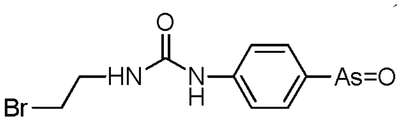,
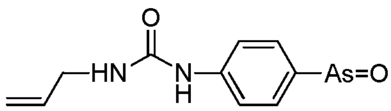,

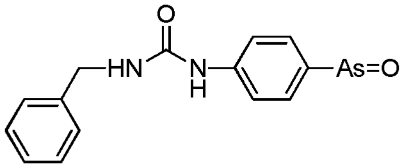,
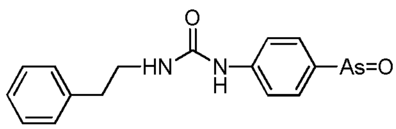,
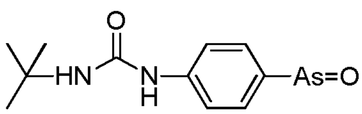,

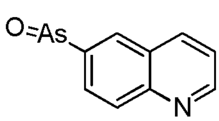,
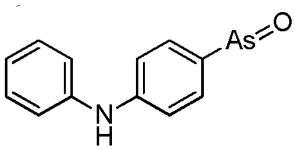,
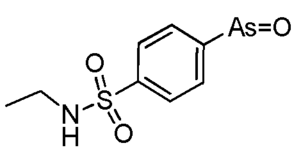,

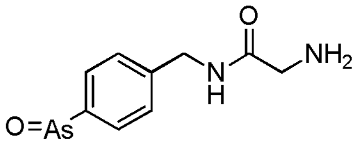,
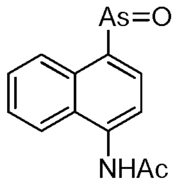,
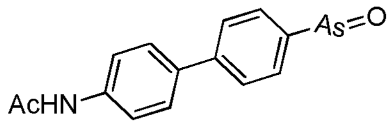,
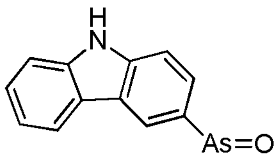,
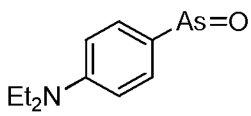,
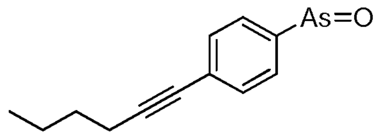,
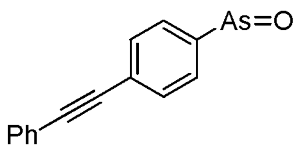,
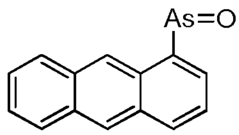,
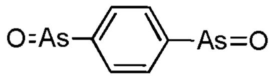,
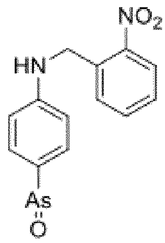,
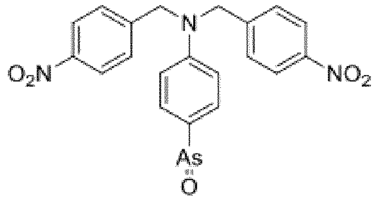,
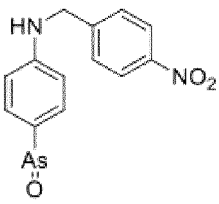,
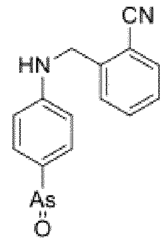,
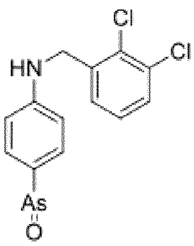,
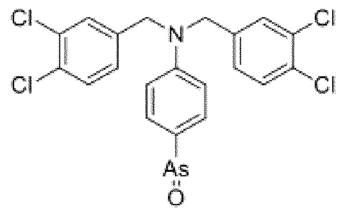
and
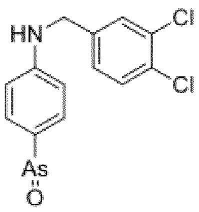.

The term "phenylarsine oxide" (PAO) as used herein refers to a small molecule compound having a specific chemical structure shown as follows:

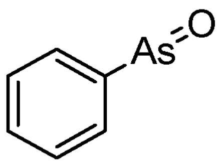.

The terms "A1" and "G1" as used herein are both small molecule compound inhibitors of PI4KIIIα protein and have relatively similar structures. The chemical structural formula of A1 is:

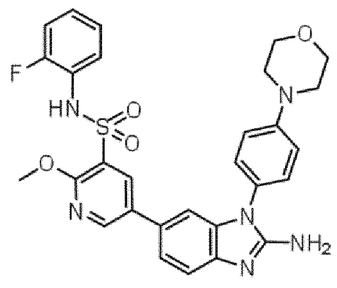

A1

.

5-(2-amino-1-(4-(4-morpholinyl)phenyl)-1*H*-benzimidazol-6-yl)-*N*-(2-fluorophenyl)-2-m ethoxy-3-pyridinesulfonamide

The chemical structural formula of G1 is:

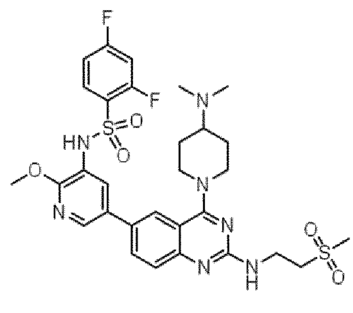

G1

.

(a*S*)-5-(2-amino-4-oxo-3-(2-(trifluoromethyl)phenyl)-3,4-dihydroquinazolin-6-yl)-*N*-(2,4-difluorophenyl)-2-methoxypyridine-3-sulfonamide.

The term "Simeprevir" ($C_{38}H_{47}N_5O_7S_2$) as used herein, is originally used for the treatment of HCV; later test proved that Simeprevir also inhibits PI4KIIIα with $IC_{50}$ of its inhibitory effect being 200 nM (Kwon J, Kim D, Park J, Park Y, Hwang Y, Wu H, Shin K, Kim I. Targeting Phosphatidylinositol 4-Kinase IIIa for Radiosensitization: A Potential Model of Drug Repositioning Using an Anti-Hepatitis C Viral Agent. Int J Radiation Oncol Biol Phys, 2016,Vol. 96 (4) pp. 867-876).

The present invention also relates to a derivative of phenylarsine oxide and an analog of A1, G1 or Simeprevir, which can also be used for treating coronavirus diseases, in particular novel coronavirus, as long as the derivative and the analog has a function of inhibiting the phosphokinase activity of PI4KIIIα protein, and a method for preparing such structural analogs have also been disclosed. In some embodiments, the derivative of phenylarsine oxide, A1, G1 or Simeprevir is an analog with a structure similar to phenylarsine oxide, A1, G1 or Simeprevir.

Coronavirus

The term "coronavirus" (CoV) as used herein refers to a group of enveloped single-stranded positive-strand RNA viruses that infect humans and a wide variety of animals, have respiratory, gastrointestinal and nervous system tropisms, cause serious illness in domestic animals and companion animals (e.g., pigs, cows, chickens, dogs, cats), and cause illness in humans ranging from the common cold to severe acute respiratory syndrome. According to the Ninth Report of the International Committee on Taxonomy of Viruses, the coronavirus is classified into four groups, α, β, γ and δ based on evolution characteristics thereof, wherein the hosts of α and β groups are mainly mammals, and γ and δ groups are found mainly in birds and avians.

Coronaviruses that can infect humans include, but are not limited to, human coronavirus 229E (HCoV-229E), NL63 (HCoV-NL63), HKU1 (HCoV-HKU1) and OC43 (HCoV-OC43) causing symptoms of upper respiratory tract infection (common cold), severe acute respiratory syndrome coronavirus (SARS-CoV) and novel coronavirus (SARS-CoV-2) causing severe respiratory diseases, and middle east respiratory syndrome coronavirus (MERS-CoV).

Coronaviruses that can infect animals include, but are not limited to, porcine transmissible gastroenteritis virus (TGEV), porcine δ-coronavirus (PDC), porcine hemagglutinating encephalomyelitis virus (PHEV), canine respiratory

coronavirus (CrCoV), mouse hepatitis virus and feline coronavirus (FCoV).

Drug administration and medical use

The term "pharmaceutically acceptable" as used herein refers to those compounds, materials, compositions, and/or dosage forms that are suitable for use in contact with human and animal tissues within the scope of reasonable medical judgment without excessive toxicity, irritation, allergic response, or other problems or complications, and are commensurate with a reasonable benefit/risk ratio. In certain embodiments, pharmaceutically acceptable compounds, materials, compositions, and/or dosage forms refer to those approved by a regulatory agency (e.g., the United States Food and Drug Administration, the National Medical Products Administration of China, or the European Medicines Agency) or listed in a generally recognized pharmacopeia (e.g., *U.S. Pharmacopeia, Chinese Pharmacopoeia,* or *European Pharmacopoeia*) for use in animals (more particularly in humans).

The term "subject" as used herein may include both humans and non-human animals. Non-human animals include all vertebrates, for example, mammals and non-mammals. The "subject" can also be livestock animals (e.g., cows, pigs, sheep, chickens, rabbits or horses), or rodents (e.g., rats or mice), or primates (e.g., gorillas or monkeys), or domestic animals (e.g., dogs or cats). The "subject" may be male or female, or may be of different age. The human "subject" may be Caucasian, African, Asian, Semitic, or other races, or a hybrid of different races. The human "subject" may be the elderly, adults, teenagers, children or infants.

In some embodiments, the subjects described herein are humans or non-human primates.

The PI4KIIIα specific inhibitors disclosed herein can be administered by an administration route well known in the art, such as by injection (e.g., subcutaneous injection, intraperitoneal injection, intravenous injection (including intravenous drip or intravenous infusion), intramuscular injection or intradermal injection) or non-injection (e.g., oral, nasal, sublingual, rectal, or topical administration). In some embodiments, the PI4KIIIα specific inhibitor described herein is administered orally, subcutaneously, intramuscularly or intravenously. In some embodiments, the PI4KIIIα specific inhibitor described herein is administered orally.

The term "therapeutically effective amount" as used herein refers to an amount of a drug that alleviates or eliminates a disease or a symptom of the subject, or that prophylactically inhibits or prevents the onset of a disease or a symptom. A therapeutically effective amount may be an amount of a drug that will alleviate to some extent one or more diseases or symptoms of the subject; an amount of a drug that can partially or completely restore to normal one or more physiological or biochemical parameters associated with the cause of a disease or a symptom; and/or an amount of a drug that may reduce the likelihood of the development of a disease or a symptom. In some embodiments, the term "therapeutically effective amount" as used herein refers to an amount of a drug that can alleviate or eliminate the coronavirus (e.g., novel coronavirus) of a subject.

The therapeutically effective dose of the PI4KIIIα specific inhibitor provided herein depends on various factors well known in the art, such as body weight, age, past medical history, current treatment underway, the health status of the object and the strength of drug interactions, allergies, hypersensitivity and side effects, as well as administration routes and disease progression degree. One skilled in the art (e.g., a physician or veterinarian) can lower or raise the dose accordingly to these or other conditions or requirements.

In some embodiments, the treatment further comprises administering a second agent to a subject in need thereof.

In some embodiments, the second agent is an agent for treating coronavirus diseases, including but not limited to Lopinavir, Ritonavir, Remdesivir, Chloroquine and Simeprevir.

In some embodiments, the PI4KIIIα specific inhibitor is administered prior to, subsequent to or concurrently with the second agent.

The present application also relates to a method for preventing or treating coronavirus diseases comprising administering an effective amount of a PI4KIIIα specific inhibitor to a subject in need thereof.

Drug screening

The present invention further provides a method for screening a drug for preventing or treating coronavirus diseases, comprising contacting a drug candidate with a PI4KIIIα protein or nucleic acid or PI4KIIIα, and detecting whether the drug candidate is capable of inhibiting the formation or activity of PI4KIIIα.

**Example 1. Preparation of Solutions of Compounds Such as Phenylarsine Oxide**

Under a low light environment, appropriate amounts of powders of compounds such as PAO (phenylarsine oxide, code No. PI01, Kaihui Pharmaceutical (Shanghai) Co., Ltd. was entrusted to synthesize the PAO), YL-05, YL-07, YL-08, YL-09, YL-10, YL-11 (BioChemPartner was entrusted to synthesize the 5 compounds having structural formula shown below), Simeprevir (code No. PI0101), PI4KIIIβ protein-specific inhibitor PIK-93 (MedChemExpress), Remdesivir

and Chloroquine phosphate were accurately weighed out by an electronic balance (purchased from METTLER TOLEDO, Switzerland) and dissolved in 5.0 mL of DMSO, and the phenylarsine oxide was stored at a concentration of 20 mM or 40 mM. The mixed solution was sterilized by filtration through a 0.22 μM filter and stored at -20°C in the dark for later use.

| Compound number | Molecular formula |
|---|---|
| PI01 | 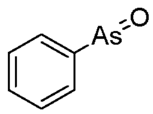 |
| YL-05 | 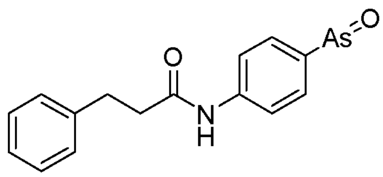 |
| YL-11 | 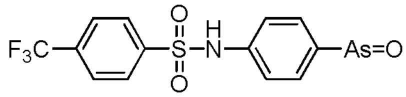 |
| YL-09 | 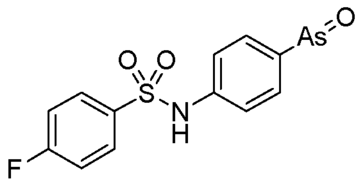 |
| YL-07 | 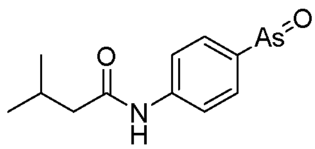 |
| YL-08 | 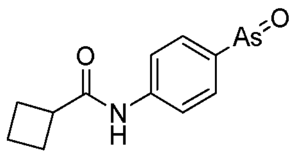 |
| YL-10 | 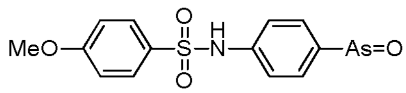 |

**Example 2. Detection of Toxicity of Phenylarsine Oxide at Different Concentrations on Vero Cells by CCK-8 Cell Proliferation-Toxicity Assay**

Vero cells (African green monkey kidney cells Vero, stored at State Key Laboratory for Diagnosis and Treatment of Infectious Diseases, Zhejiang University) were seeded at 5000 cells/well in a 96-well culture plate (purchased from Corning Inc.), and cultured in an incubator at 37°C with 5% $CO_2$ (Thermo 3110, purchased from Thermo Electron Corporation) to a cell monolayer; the culture media was discarded, and the cells were washed 2 times with Hank's solution.

Drug solution dilution: In the first round of experiment, an MEM medium (purchased from Life Technologies) was used to dilute phenylarsine oxide to obtain serial concentrations of phenylarsine oxide, and the initial concentration was 200 nM and 7 serial two-fold dilutions were made to 1.56 nM. 2 replicate wells were set for each phenylarsine oxide concentration, and 150 μL of drug solution was added into each well; meanwhile, Vero cell normal growth control was set, and culture was performed in an incubator at 37°C with 5% $CO_2$ for 48 hours. In the second round of experiment, phenylarsine oxide and PIK-93 had an initial concentration of 800 nM, respectivelly, and 7 serial two-fold dilutions were made to 6.25 nM; Remdesivir had an initial concentration of 300 μM, and 6 serial three-fold dilutions were made to 0.4 μM; and Chloroquine phosphate had an initial concentration of 300 μM and 6 serial three-fold dilutions were made to

0.4 μM. PIK-93 had an initial concentration of 800 nM and 7 serial two-fold dilutions were made to 6.25 nM. 2 replicate wells were set for each drug concentration, and 150 μL of drug solution was added into each well; meanwhile, Vero cell normal growth control was set, and culture was performed in an incubator at 37°C with 5% $CO_2$ for 48 h.

10 μL or 15 μL of CCK-8 reagent (cell proliferation-toxicity assay kit (CCK-8) (purchased from Dojindo Molecular Technologies, Inc., Japan)) was added to each well, and the OD value at 450 nm was measured using a microplate reader (Bio-Rad 680, purchased from Bio-Rad Laboratories, Inc., USA) after 3 hours; the dose-toxic effect and maximum non-toxic concentration of phenylarsine oxide on cells were calculated, and a phenylarsine oxide-cytotoxicity response curve was plotted, providing a basis for selecting the optimum test concentration for antiviral efficacy *in vitro.*

The results for the first round of test showed that phenylarsine oxide with a concentration of 200 nM and above had a significant toxic effect on Vero cells, and the cell death reached 50%, i.e., $CC_{50}$ > 200 nM. Phenylarsine oxide with a concentration below 100 nM had no significant toxic effect on cells, and cell viability was greater than 90% (see Table 1A and FIG. 1A).

Table 1A: Toxic effect of phenylarsine oxide at different concentrations on Vero cells

| Phenylarsine oxide concentration nM | 200 | 100 | 50 | 25 | 12.5 | 6.25 | 3.12 | 1.56 | Undosed control | |
|---|---|---|---|---|---|---|---|---|---|---|
| $A_{450}$ nm value | 1.00 | 1.71 | 1.90 | 1.80 | 1.75 | 1.79 | 1.75 | 1.77 | 1.85 | 1.94 |
| | 0.68 | 1.86 | 1.98 | 1.95 | 1.95 | 1.86 | 2.06 | 1.94 | 1.86 | 1.99 |
| Cell viability % | 50% | cell viability greater than 90% and no significant cytotoxicity | | | | | | | 100% | |

The results for the second round of test showed that phenylarsine oxide (PI01) with a concentration of 400 nM and above had a significant toxic effect on Vero cells, the cell death rate reached 50% and above, and $CC_{50}$ was 380 nM. The drug with a concentration of 100 nM and below had no significant toxic effect on cells, the cell viability was greater than 90%, and $CC_{10}$ was 100 nM.

PIK-93, a PI4KIIIβ protein-specific inhibitor, at a concentration of 800 nM and below had no significant toxic effect on Vero cells.

The control drug, Remdesivir, had $CC_{50}$ of 208 μM on Vero cells, the drug at a concentration of 50 μM and below had no significant toxic effect on cells, the cell viability was greater than 90%, and $CC_{10}$ was 55 μM.

The control drug Chloroquine phosphate had $CC_{50}$ of about 250 μM on Vero cells, and $CC_{10}$ was 120 μM.

The specific results are shown in [Tables 1B, C, D and FIGs. 1B, C, D].

Table 1B: Toxic effect of drug phenylarsine oxide at different concentrations on Vero cells

| Phenylarsine oxide concentration μM | 800 | 400 | 200 | 100 | 50 | 25 | 12.5 | 6.25 | Undosed control |
|---|---|---|---|---|---|---|---|---|---|
| A450 nm value | 0.36 | 0.93 | 1.36 | 1.93 | 2.05 | 2.05 | 2.04 | 2.05 | 2.03 |
| | 0.44 | 0.89 | 1.56 | 1.84 | 1.99 | 2.00 | 1.93 | 2.04 | 2.00 |

Table 1C: Toxic effect of drug Remdesivir at different concentrations on Vero cells

| Remdesivir concentration μM | 300 | 100 | 33.3 | 11.1 | 3.7 | 1.2 | 0.4 | Undosed control |
|---|---|---|---|---|---|---|---|---|
| A450 nm value | 0.47 | 1.62 | 1.85 | 1.97 | 1.98 | 2.01 | 2.03 | 2.03 |
| | 0.44 | 1.60 | 1.96 | 1.94 | 1.99 | 1.99 | 2.04 | 2.00 |

Table ID: Toxic effect of drug Chloroquine phosphate at different concentrations on Vero cells

| Chloroquine concentration uM | 300 | 100 | 33.3 | 11.1 | 3.7 | 1.2 | 0.4 | Undosed control |
|---|---|---|---|---|---|---|---|---|
| A450 nm value | 0.59 | 1.98 | 2.01 | 2.00 | 2.05 | 2.06 | 2.04 | 2.03 |
| | 0.63 | 1.95 | 2.00 | 2.04 | 2.05 | 2.02 | 2.05 | 2.00 |

**Example 3. Determination of Novel Coronavirus $TCID_{50}$ by Microcytopathic Observation Method**

The Vero cells were seeded in a 96-well culture plate at a concentration of 10,000 cells/well, cultured in an incubator at 37°C with 5% $CO_2$ until confluence of 75% to 90% cells at the most vigorous logarithmic growth phase, the cell culture media was discarded, and the cells were washed 2 times with Hank's solution (to remove residual bovine serum).

The novel coronavirus was obtained by separating from sputum samples of clinical infected patients, verified by whole-gene sequencing, and then stored at State Key Laboratory for Diagnosis and Treatment of Infectious Diseases, Zhejiang University. The experiment was carried out in a biosafety shelter laboratory-3 (BSL-3: laboratory accreditation number: CNAS BL002, State Key Laboratory for Diagnosis and Treatment of Infectious Diseases, Zhejiang University).

The novel coronavirus strain was diluted 1000-fold with a virus growth medium (500 mL MEM medium containing 2% FBS, 100 U/mL of penicillin, 100 μg/mL of streptomycin, and 16 μg/mL of TPCK-trypsin) (all purchased from Life Technologies Corporation) as an initial concentration that was then serially 10-fold diluted to make 8 serial concentrations. Each dilution of virus cells was inoculated in 4 wells at 100 μl/well, cell normal growth control was set, and the cells were cultured in an incubator at 37°C with 5% $CO_2$ for 6 days.

The change in cell morphology and cytopathic effect (CPE) was observed daily under an inverted microscope; with less than 25% change in cell morphology CPE as "+", 26% to 50% change in cell morphology CPE as "++", 51% to 75% change in cell morphology CPE as "+++", and 76% to 100% change in cell morphology CPE as "++++", the virus median infective concentration $TCID_{50}$ was calculated by the Reed-Muench method.

The result showed that the $TCID_{50}$ of the new coronavirus used in the experiment was $10^{-6.5}$/100 μL.

**Example 4. *In Vitro* Inhibitory Effect of Phenylarsine Oxide on Novel Coronavirus (SARS-Cov-2)**

Cell culture: The Vero cells were seeded in a 24-well plate at a concentration of 50,000 cells/1 mL/well, cultured in an incubator at 37°C with 5% $CO_2$ until confluence of 75% to 90% cells at the most vigorous logarithmic growth phase, the cell culture media was discarded, and the cells were washed 2 times with Hank's solution.

Drug solution formulation: Stock concentrations of drugs were diluted in the medium to the following final concentrations: in the first round of experiment, phenylarsine oxide had an initial concentration of 200 nM and 7 serial two-fold dilutions were made to 1.56 nM; in the second round of experiment, phenylarsine oxide and PIK-93 had an initial concentration of 800 nM and 7 serial two-fold dilutions were made to 6.25 nM; Remdesivir had an initial concentration of 100 μM, and 6 serial three-fold dilutions were made to 0.4 μM; and Chloroquine phosphate had an initial concentration of 100 μM and 6 serial three-fold dilutions were made to 0.3 μM.

Virus solution formulation: The novel coronavirus (SARS-CoV-2 strain BetaCov/Wuhan/IME-BJ01/2020 (GWHACBBO1000000)) was diluted in the medium until the final concentration of infected cells was 100 $TCID_{50}$.

Infection of Vero cells by virus: The Vero cells were infected with 250 μL/well of the formulated virus (100 $TCID_{50}$), and virus-infected cell alone control and normal cell control were set. The cells were allowed for adsorption in an incubator at 37°C with 5% $CO_2$ for 3 hours, the virus-containing culture media was discarded, and the cells were washed 2 times with Hank's solution.

Drug treatment: The formulated culture media containing drugs with different concentrations were added to the Vero cell culture plate that was pre-infected with virus at a concentration of 1 mL/well, wherein 2 replicate wells were set for each phenylarsine oxide concentration. The cells were cultured in an incubator at 37°C with 5% $CO_2$ for 48 hours.

The novel coronavirus nucleic acid was detected by a fluorescence-based quantitative PCR method. 200 μL of culture supernatant was pipetted, virus nucleic acid was extracted by a magnetic bead method nucleic acid extraction kit (MVR01) and a full-automatic nucleic acid extractor (EX3600, Shanghai ZJ Bio-Tech Co., Ltd.), and the final elution volume was 50 μl. 5 μL of nucleic acid extract was taken, and the virus nucleic acid level was detected by employing a one-step novel coronavirus nucleic acid detection kit (a fluorescence PCR method, Cat. # Z-RR-0479-02-50, certificate number 20203400057, purchased from Shanghai ZJ Bio-Tech Co., Ltd.). The results were expressed as Ct values for virus level. The fluorescence PCR method was applied, and the corresponding relation between the Ct value and the virus copy number was as follows: $Y = -3.33x + 48.69$, where y is the Ct value, and x is the log of the number of viruses with base 10.

The results for the first round of experiment showed that phenylarsine oxide at a concentration of 20-100 nM had a significant antiviral effect on a Vero cell model, and $EC_{50}$ = 20 nM (see Table 2A and FIG. 2A). FAM Ct values in Table 2 represented viral nucleic acid levels. The larger Ct value indicates less viruses. Conversely, the smaller Ct value indicates more viruses.

The results for the second round of experiment showed that $EC_{50}$ of drug PI01 for inhibiting the novel coronavirus in the Vero cell model was 19.2 nM. $EC_{50}$ of Remdesivir for inhibiting the novel coronavirus was 0.52 μM. $EC_{50}$ of Chloroquine phosphate for inhibiting the novel coronavirus was 50 μM. PIk-93 at a concentration of 800 nM and below had no significant inhibitory effect on the novel coronavirus. Specific results are shown in Tables 2B, C, D and FIGs.

2B, C, D.

In addition, the inhibitory effect of PI0101 on the novel coronavirus was also assayed. Cells were seeded into a 96-well test plates at a density of 10,000 cells/well and cultured overnight in an incubator at 37°C with 5% $CO_2$. Next day, 3-fold diluted PI0101 (with an initial concentration of 10 μM, 8 concentration points, two duplicate wells) were added followed by virus addition to cells at 100 $TCID_{50}$/well. Cell control (cells, no compound treatment or viral infection), virus control (cells infected with virus, no compound treatment) and media control (media only) were set, and the above were cultured in an incubator at 37°C with 5% $CO_2$ for 76 hours. It could be found from the results that PI0101 had $EC_{50}$ of 3.85 μM and $CC_{50}$ greater than 10 μM.

Table 2A: *In vitro* inhibitory effect of phenylarsine oxide at different concentrations on novel coronavirus

| Name | Concentration nM | FAM Ct value | Antiviral effective concentration on *in vitro* cell model |
|---|---|---|---|
| Phenyl arsine oxide | 200 | Cytotoxi city | $CC_{50}$ > 200 nM |
| | 200 | | |
| | 100 | 26.4 | Phenylarsine oxide at a concentration of 20-100 nM had a significant antiviral effect on cell model, and $EC_{50}$ = 20 nM |
| | 100 | 28.32 | |
| | 50 | 22.91 | |
| | 50 | 22.88 | |
| | 25 | 20.35 | |
| | 25 | 20.02 | |
| | 12.5 | 18.57 | |
| | 12.5 | 18.75 | |
| | 6.25 | 18.59 | |
| | 6.25 | 18.37 | |
| | 3.12 | 18.32 | |
| | 3.12 | 18.34 | |
| | 1.56 | 19.02 | |
| | 1.56 | 18.28 | |
| Viral infection control without phenyl arsine oxide | | 18.71 | |
| | | 18.59 | |

Table 2B: *In vitro* inhibitory effect of phenylarsine oxide at different concentrations on novel coronavirus

| Drug number | Drug name | Drug concentration nM | FAM Ct value |
|---|---|---|---|
| 1 | Phenylarsine oxide | 800 | 31.52 |
| | | 800 | 28.87 |
| | Phenylarsine oxide | 400 | 27.19 |
| | | 400 | 26.64 |
| | Phenylarsine oxide | 200 | 27.51 |
| | | 200 | 29.48 |
| | Phenylarsine oxide | 100 | 20.31 |
| | | 100 | 19.24 |
| | Phenylarsine oxide | 50 | 17.41 |
| | | 50 | 17.19 |
| | Phenylarsine oxide | 25 | 15.94 |
| | | 25 | 16.51 |
| | Phenylarsine oxide | 12.5 | 15.36 |
| | | 12.5 | 15.38 |
| | Phenylarsine oxide | 6.25 | 14.79 |
| | | 6.25 | 14.96 |
| | Viral infection control without drug | | 14.18 |
| | | | 15.38 |

Table 2C: *In vitro* inhibitory effect of Remdesivir at different concentrations on novel coronavirus

| Drug number | Drug name | Drug concentration μM | FAM Ct value |
|---|---|---|---|
| 2 | Remdesivir | 100 | 29.52 |
| | | 100 | 29.88 |
| | Remdesivir | 33.3 | 27.92 |
| | | 33.3 | 29.46 |
| | Remdesivir | 11.1 | 28.18 |
| | | 11.1 | 27.86 |
| | Remdesivir | 3.7 | 23.72 |
| | | 3.7 | 24.15 |
| | Remdesivir | 1.2 | 18.52 |
| | | 1.2 | 18.55 |
| | Remdesivir | 0.4 | 14.73 |
| | | 0.4 | 14.98 |
| | Viral infection control without drug | | 14.18 |
| | | | 15.38 |

Table 2D: *In vitro* inhibitory effect of Chloroquine phosphate (Chloroquine) at different concentrations on novel coronavirus

| Drug number | Drug name | Drug concentration μM | FAM Ct value |
|---|---|---|---|
| 3 | Chloroquine | 100 | 20.43 |
| | | 100 | 19.17 |
| | Chloroquine | 33.3 | 15.34 |
| | | 33.3 | 14.81 |
| | Chloroquine | 11.1 | 14.6 |
| | | 11.1 | 14.5 |
| | Chloroquine | 3.7 | 13.99 |
| | | 3.7 | 14.8 |
| | Chloroquine | 1.2 | 13.77 |
| | | 1.2 | 14.76 |
| | Chloroquine | 0.4 | 14.67 |
| | | 0.4 | 13.91 |
| | Viral infection control without drug | | 14.18 |
| | | | 15.38 |

**Example 5. Assay of the Activity of Phenylarsine Oxide Against Human Coronavirus (HCoV) 229E *In Vitro* by Cytopathic Effect (CPE) Experiment**

Drug solution formulation: Stock concentrations of drugs were diluted in the medium to the following final concentrations. In the first round of experiment, the initial concentrations for phenylarsine oxide and compounds YL-05, YL-07, YL-08, YL-09, YL-10 and YL-11 to have toxicity on MRC5 cells and inhibition on HCoV 229E (human coronavirus type 229E, purchased from ATCC) were observed to be 800 nM and 200 nM, respectively, and 7 serial two-fold dilutions were made to 6.25 nM and 1.56 nM, respectively; and the initial concentrations for Remdesivir to have toxicity on MRC5 cells and inhibition on HCoV 229E were 100 μM and 1000 nM, respectively, and 7 serial three-fold dilutions were made to 0.41 μM and 0.46 nM, respectively.

In the second round of experiment, the initial concentrations for phenylarsine oxide to have cytotoxicity and viral inhibition were observed to be 800 nM and 400 nM, respectively, and 7 serial two-fold dilutions were made to 6.25 nM and 3.13 nM, respectively; and the initial concentrations for compounds YL-07, YL-08, YL-09, YL-10 and YL-11 to have toxicity on MRC5 cells and inhibition on HCoV 229E were observed to be 40 μM, and 7 serial three-fold dilutions were made to 0.02 μM; the initial concentrations for Remdesivir to have toxicity on cells and inhibitory effect on viruses were 100 μM and 1000 nM, respectively, and 7 serial three-fold dilutions were made to 0.41 μM and 0.46 nM, respectively.

MRC5 cells and HCoV 229E strain were purchased from ATCC. The cells were cultured in EMEM (Sigma) medium supplemented with 10% fetal bovine serum (Hyclone), 1% double antibody (Hyclone), 1% L-glutamine (Gibco) and 1% non-essential amino acid (Gibco). The experimental culture medium was EMEM (Sigma) medium supplemented with 5% fetal bovine serum (Hyclone), 1% double antibody (Hyclone), 1% L-glutamine (Gibco) and 1% non-essential amino acid (Gibco).

MRC5 cells were seeded into a 96-well test plate at a density of 20,000 cells/well and cultured overnight in an incubator at 37°C with 5% $CO_2$. Next day, double-diluted phenylarsine oxide (8 concentration points, two duplicate wells) were added followed by virus addition to cells at 200 $TCID_{50}$/well. Cell control (cells, no compound treatment or viral infection), virus control (cells infected with virus, no compound treatment) and media control (media only) were set. The final concentration of DMSO in the culture medium was 0.5%. The cells were cultured in an incubator for 3 days. Cell viability was assayed using the cell viability assay kit CellTiter Glo (Promega). The cytotoxicity experiment had the same conditions as the antiviral experiment, but no virus infection. The antiviral activity and cytotoxicity of phenylarsine oxide were represented by the inhibition rate (%) of the compound at different concentrations against virus-induced cytopathic effect and the viability (%) of MRC5 cells, respectively. The calculation formula is as follows:

Inhibition rate (%) = (test well reading - mean of viral control)/(mean of cell control - mean of viral control) × 100

Cell viability (%) = (test well reading - mean of culture medium control)/(mean of cell control - mean of culture medium control) × 100

The nonlinear fitting analysis of the inhibition rate and cell viability of phenylarsine oxide was performed using GraphPad Prism (version 5) and the median effective concentrations ($EC_{50}$) of phenylarsine oxide was calculated.

The results for the first round showed that the $EC_{50}$ value of phenylarsine oxide was 55.35 nM, and the $CC_{50}$ value was 256.8 nM; the $EC_{50}$ value of Remdesivir was 26.42 nM, and the $CC_{50}$ value was greater than 100 μM; the $EC_{50}$ values of compounds YL-05, YL-07, YL-08, YL-09, YL-10 and YL-11 were all greater than 200 nM, and the $CC_{50}$ values were all greater than 800 nM; and the specific results are shown in FIG. 3A and Table 3. The results for the second round showed that the $EC_{50}$ value of phenylarsine oxide was 37.03 nM, and the $CC_{50}$ value was 68.92 nM; the $EC_{50}$ value of Remdesivir was 13.11 nM, and the $CC_{50}$ value was 30.66 μM; the $EC_{50}$ values of compounds YL-07, YL-08 and YL-09 were all greater than 40 μM, and the $CC_{50}$ values were 6.24 μM, 7.64 μM and 13.88 μM, respectively; the $EC_{50}$ values of compounds YL-10 and YL-11 were 3.95 μM and 2.1 μM, respectively, and the $CC_{50}$ values were 14 μM and 6.37 μM, respectively; and The specific results are shown in FIG. 3B and Table 3. the $EC_{50}$ of PI0101 (Simeprevir) was 3.41 μM, and the $CC_{50}$ was 8.46 μM.

**Table 3. Results of test for compounds**

| No. | Compounds | The first test | | | The second test | | |
|---|---|---|---|---|---|---|---|
| | | $EC_{50}$ | $CC_{50}$ | SI ($CC_{50}/EC_{50}$) | $EC_{50}$ | $CC_{50}$ | SI ($CC_{50}/EC_{50}$) |
| 1 | PI01 | 55.35 nM | 256.8 nM | 4.64 | 37.03 nM | 68.92 nM | 1.86 |
| 2 | YL-05 | > 200 nM | > 800 nM | NA | ND | ND | ND |
| 3 | YL-07 | > 200 nM | > 800 nM | NA | > 40 μM | 6.24 μM | NA |
| 4 | YL-08 | > 200 nM | > 800 nM | NA | > 40 μM | 7.64 μM | NA |
| 5 | YL-09 | > 200 nM | > 800 nM | NA | > 40 μM | 13.88 μM | NA |
| 6 | YL010 | > 200 nM | > 800 nM | NA | 3.95 μM | 14.00 μM | 3.55 |
| 7 | YL011 | > 200 nM | > 800 nM | NA | 2.10 μM | 6.37 μM | 3.03 |
| 8 | PI0101 | ND | ND | ND | 3.41 μM | 8.46 μM | 2.48 |
| 9 | Remdesivir | 26.42 μM | > 100 μM | > 3785.01 | 13.11 μM | 30.66 μM | 2338.60 |
| Notes: ND = Not Detected | | | | | | | |

It will be apparent to those skilled in the art that, although specific embodiments of the present invention have been described herein for purposes of illustration, various modifications may be made without deviating from the spirit and scope of the present invention. Therefore, the detailed description of embodiments and examples of the present invention should not be construed as limiting the scope of the present invention. The present invention is not limited except as by the appended claims. All documents cited herein are incorporated by reference in their entirety.

## Claims

1. Use of a PI4KIIIα specific inhibitor in the preparation of a drug for preventing or treating coronavirus diseases.

2. The use according to claim 1, wherein the PI4KIIIα specific inhibitor is an antibody, a small molecule compound, an RNAi molecule or an antisense nucleic acid.

3. The use according to claim 2, wherein the antibody is a monoclonal antibody or a polyclonal antibody.

**4.** The use according to claim 3, wherein the antibody is a chimeric antibody, a humanized antibody or a fully human antibody.

**5.** The use according to claim 2, wherein the RNAi molecule is a small interference RNA (siRNA), a short hairpin RNA (shRNA) or a microRNA (miRNA).

**6.** The use according to claim 2 or 5, wherein the RNAi molecule is 18-100 bases in length.

**7.** The use according to any one of claims 2 and 5-6, wherein the RNAi molecule is modified to enhance its stability.

**8.** The use according to claim 2, wherein the PI4KIIIα specific inhibitor is a small molecule compound.

**9.** The use according to claim 8, wherein the small molecule compound is phenylarsine oxide or a derivative thereof, G1 and an analog thereof, A1 and an analog thereof, or Simeprevir and an analog thereof.

**10.** The use according to claim 9, wherein the phenylarsine oxide and the derivative thereof have a structure as represented by formula (I) or a pharmaceutically acceptable salt thereof,

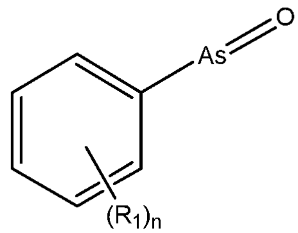

Formula (I)

wherein each $R_1$ is independently selected from (a) H, halogen, nitro, cyano, hydroxy, amino, carbamoyl, $C_{1-6}$ alkyl, $C_{2-6}$ alkynyl, $C_{2-6}$ alkenyl, $C_{1-6}$ alkoxy, $C_{1-6}$ haloalkyl, $C_{1-6}$ alkylene-$NH_2$, $C_{1-6}$ alkylene-NH-C(O)H, -As(O), -N=NH, N-($C_{1-6}$ alkyl)amino, *N*,*N*-($C_{1-6}$ alkyl)$_2$ amino, -NH-C(O)H, -NH-S(O)$_2$H, -C(O)OH, -OC(O)H, -SH, -S(O)$_2$H, -S(O)$_2$-$NH_2$ or heterocyclyl, and optionally substituted with $R_2$ or $R_3$, wherein the $R_2$ and the $R_3$ are each independently selected from amino, $C_{1-6}$ alkyl, $C_{1-6}$ alkoxy, $C_{1-6}$ haloalkyl, *N*-($C_{1-6}$ alkyl) amino, *N*-(6-12 membered aryl)amino, *N*,*N*-($C_{1-6}$ alkyl)$_2$ amino, $C_{3-6}$ cycloalkyl, 6-12 membered aryl or 3-12 membered heterocyclyl, and optionally substituted with one or more halogen, nitro, cyano, hydroxy, amino, carbamoyl, -NH-C(O)-$R_5$, -C(O)O$R_4$, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, $R_4$ is $C_{1-6}$ alkyl, and optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, and $R_5$ is selected from H, $C_{1-6}$ alkyl, $C_{2-6}$ alkynyl, $C_{2-6}$ alkenyl, $C_{1-6}$ alkoxy or $C_{1-6}$ haloalkyl, and/or

(b) $R_1$ on two adjacent carbon atoms forms 5-12 membered cycloalkyl, aryl or heterocyclyl, and is optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, wherein n is an integer from 0 to 5.

**11.** The use according to claim 10, wherein n is an integer from 0 to 2, and each of the $R_1$ is independently selected from H, halogen, nitro, cyano, hydroxy, amino, carbamoyl, $C_{1-6}$ alkyl, $C_{1-6}$ alkoxy, $C_{1-6}$ haloalkyl, -As(O), *N*-($C_{1-6}$ alkyl)amino, *N*,*N*-($C_{1-6}$ alkyl)$_2$ amino,-NH-C(O)H or -NH-S(O)$_2$H, and optionally substituted with the $R_2$ or the $R_3$.

**12.** The use according to claim 10, wherein n is an integer from 0 to 2, and each of the $R_1$ is independently selected from H, halogen, nitro, cyano, hydroxy, amino, $C_{1-6}$ alkyl, $C_{1-6}$ alkoxy, $C_{1-6}$ haloalkyl, -As(O), -NH-C(O)H or -NH-S(O)$_2$H, and optionally substituted with the $R_2$ or the $R_3$.

**13.** The use according to claim 10, wherein n is 1 or 2, each of the $R_1$ is independently selected from H, halogen, amino, $C_{1-6}$ alkoxy, $C_{1-6}$ haloalkyl, -NH-C(O)$R_2$ or-NH-S(O)$_2R_3$, wherein the $R_2$ is $C_{1-6}$ alkyl and optionally substituted with one 6-12 membered aryl, and the $R_3$ is 6-12 membered aryl and optionally substituted with one halogen, $C_{1-6}$ alkoxy or $C_{1-6}$ haloalkyl.

**14.** The use according to claim 13, wherein the $R_1$ is located at ortho- and/or para-position of -As(O).

**15.** The use according to claim 10, wherein n is 0.

**16.** The use according to claim 9, wherein the small molecule compound is selected from the group consisting of the following compounds:

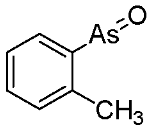 , 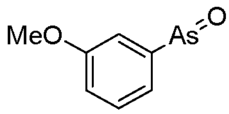 , 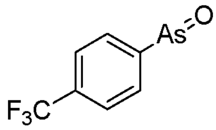 , 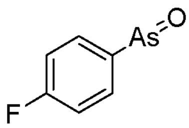 , 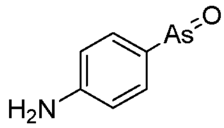 ,

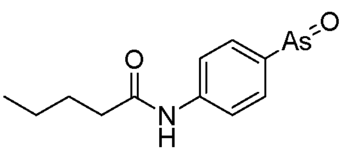 , 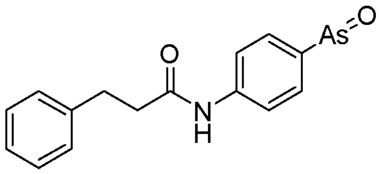 , 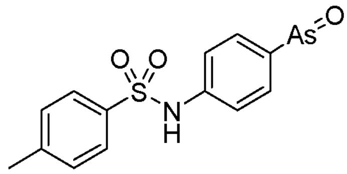 ,

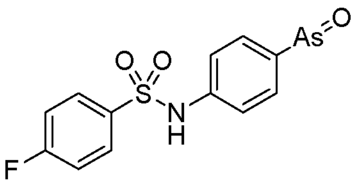 , 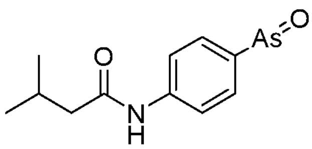 , 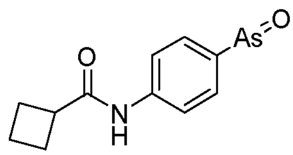 ,

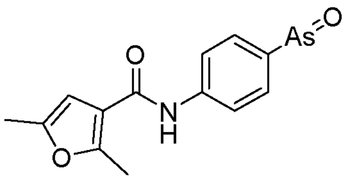 , 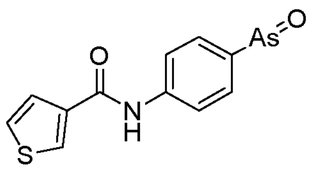 , 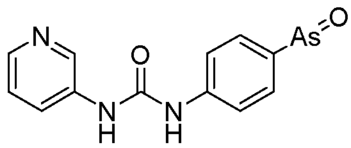 ,

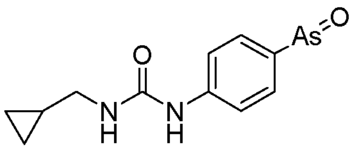 , 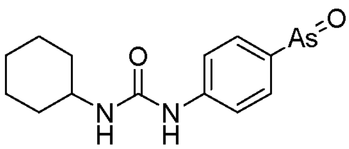 , 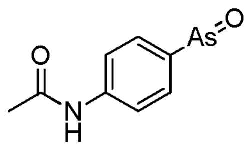 ,

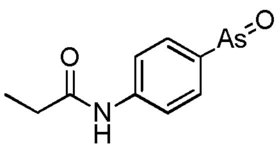 , 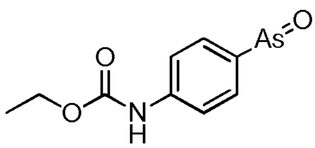 , 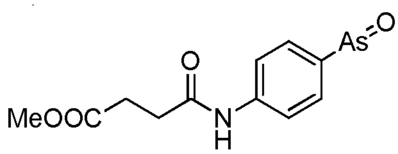 ,

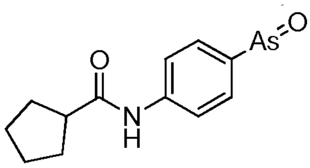 , 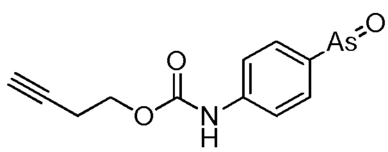 , 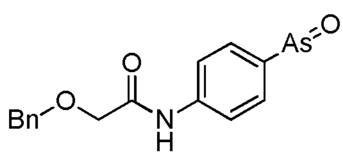 ,

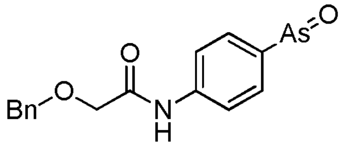,
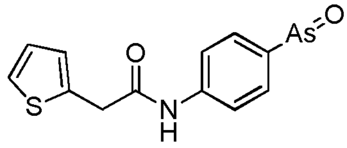,
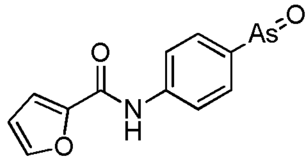,

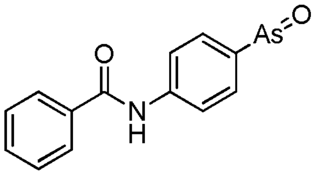,
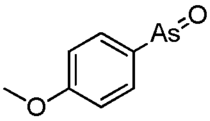,
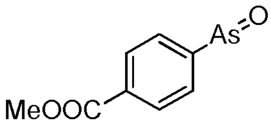,
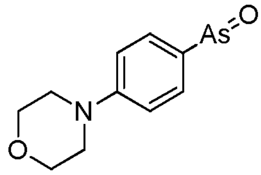,

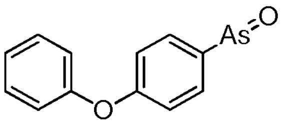,
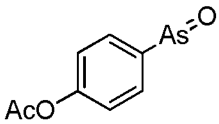,
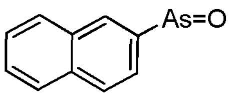,
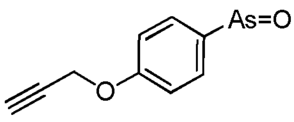,

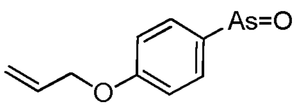,
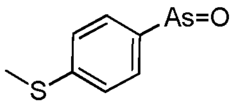,
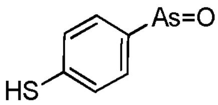,
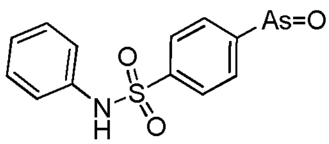,

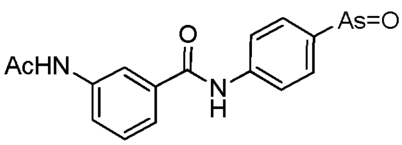,
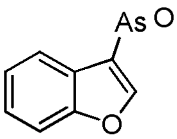,
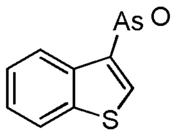,
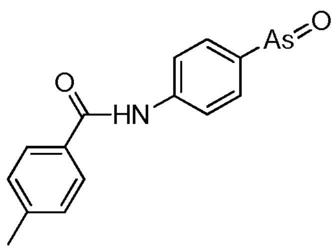,

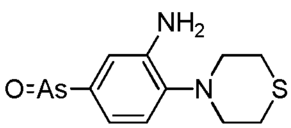,
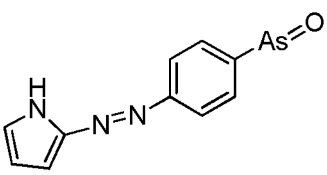,
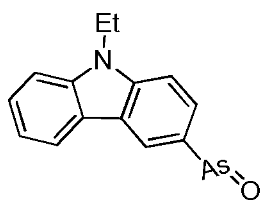,

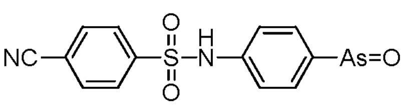,
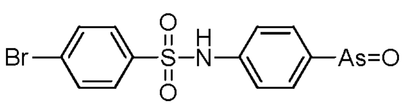,

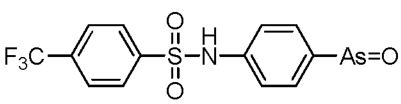,
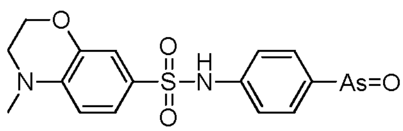,
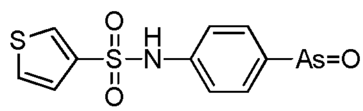,

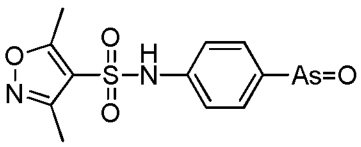,
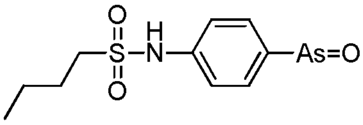,
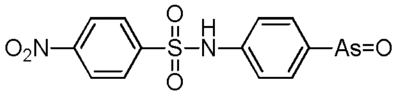,

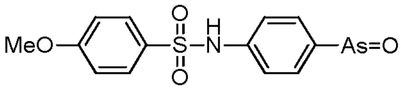,
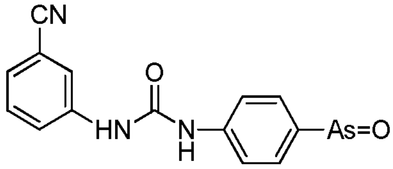,

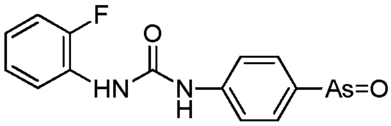,
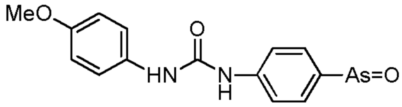,

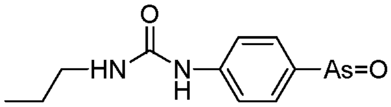,
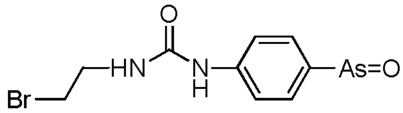,

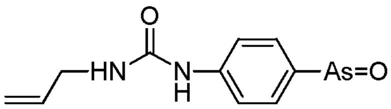,
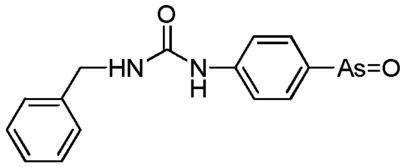,

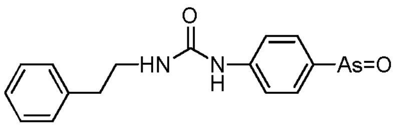,
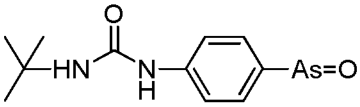,
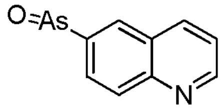,

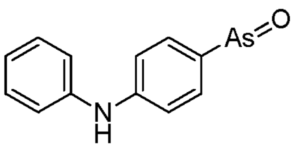,
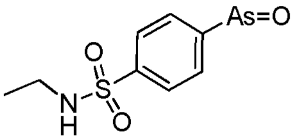,
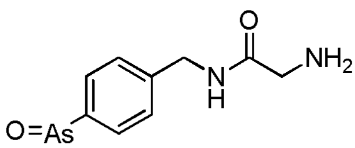,
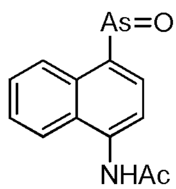,

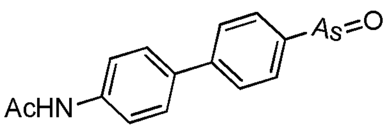,
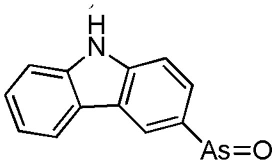,
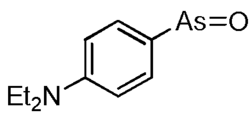,

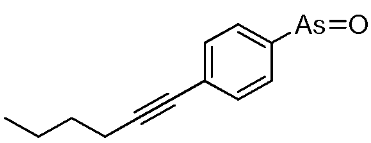,
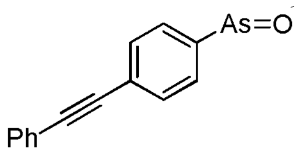,
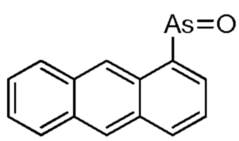,

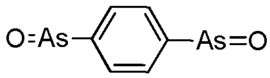, 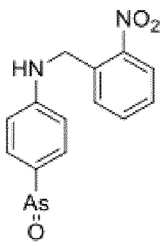, 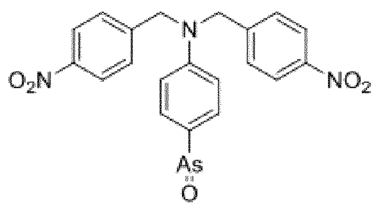, 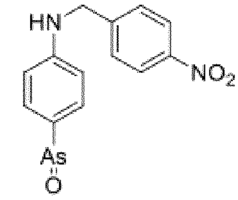,

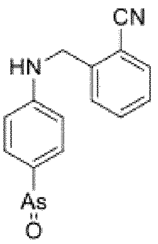, 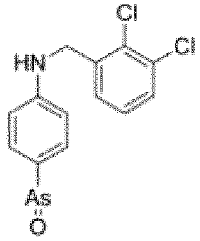, 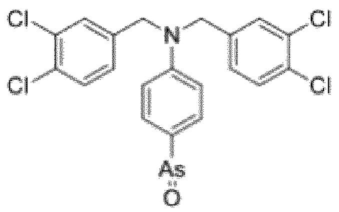 and 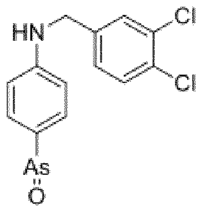.

**17.** The use according to claim 1, wherein the subject is a human or a mammal.

**18.** The use according to claim 1, wherein the coronavirus is a novel coronavirus.

**19.** The use according to claim 1, wherein the coronavirus is chicken infectious bronchitis virus, porcine epidemic diarrhea virus, porcine transmissible gastroenteritis virus, porcine hemagglutinating encephalomyelitis virus, porcine δ-coronavirus, canine respiratory coronavirus, mouse hepatitis virus, feline coronavirus, human coronavirus, severe acute respiratory syndrome virus and middle east respiratory syndrome virus.

**20.** The use according to claim 1, further comprising administering a second agent to a subject in need thereof.

**21.** The use according to claim 20, wherein the second agent is an agent for treating coronavirus diseases.

**22.** The use according to claim 21, wherein the PI4KIIIα specific inhibitor is administered prior to, subsequent to or concurrently with the second agent.

**23.** A method for screening a drug for preventing or treating coronavirus diseases, comprising contacting a drug candidate with a PI4KIIIα protein or nucleic acid or PI4KIIIα, and detecting whether the drug candidate is capable of inhibiting the formation or activity of PI4KIIIα.

**24.** Use of the compound of formula (I) in the preparation of a drug for preventing or treating coronavirus diseases:

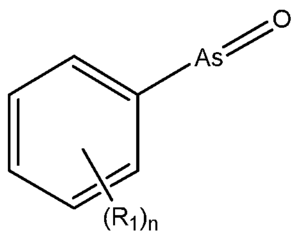

Formula (I)

wherein each $R_1$ is independently selected from (a) H, halogen, nitro, cyano, hydroxy, amino, carbamoyl, $C_{1-6}$ alkyl, $C_{2-6}$ alkynyl, $C_{2-6}$ alkenyl, $C_{1-6}$ alkoxy, $C_{1-6}$ haloalkyl, $C_{1-6}$ alkylene-$NH_2$, $C_{1-6}$ alkylene-NH-C(O)H, -As(O), -N=NH, N-($C_{1-6}$ alkyl)amino, *N*,*N*-($C_{1-6}$ alkyl)$_2$ amino, -NH-C(O)H, -NH-S(O)$_2$H, -C(O)OH, -OC(O)H, -SH, -S(O)$_2$H, -S(O)$_2$-$NH_2$ or heterocyclyl, and optionally substituted with $R_2$ or $R_3$, wherein the $R_2$ and the $R_3$ are each independently selected from amino, $C_{1-6}$ alkyl, $C_{1-6}$ alkoxy, $C_{1-6}$ haloalkyl, *N*-($C_{1-6}$ alkyl) amino, *N*-(6-12

membered aryl)amino, *N*,*N*-($C_{1-6}$ alkyl)$_2$ amino, $C_{3-6}$ cycloalkyl, 6-12 membered aryl or 3-12 membered heterocyclyl, and optionally substituted with one or more halogen, nitro, cyano, hydroxy, amino, carbamoyl, -NH-C(O)-$R_5$, -C(O)O$R_4$, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, $R_4$ is $C_{1-6}$ alkyl, and optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-, and $R_5$ is selected from H, $C_{1-6}$ alkyl, $C_{2-6}$ alkynyl, $C_{2-6}$ alkenyl, $C_{1-6}$ alkoxy or $C_{1-6}$ haloalkyl, and/or
(b) $R_1$ on two adjacent carbon atoms forms 5-12 membered cycloalkyl, aryl or heterocyclyl, and is optionally substituted with one or more halogen, nitro, cyano, hydroxy, amino, carbamoyl, 6-12 membered aryl, $C_{1-6}$ alkyl, $C_{2-6}$ alkynyl, $C_{2-6}$ alkenyl, $C_{1-6}$ alkoxy, $C_{1-6}$ haloalkyl, 3-6 membered heterocyclyl, $C_{3-6}$ cycloalkyl or Bn-O-,
wherein n is an integer from 0 to 5.

**25.** The use according to claim 24, wherein the compound is phenylarsine oxide.

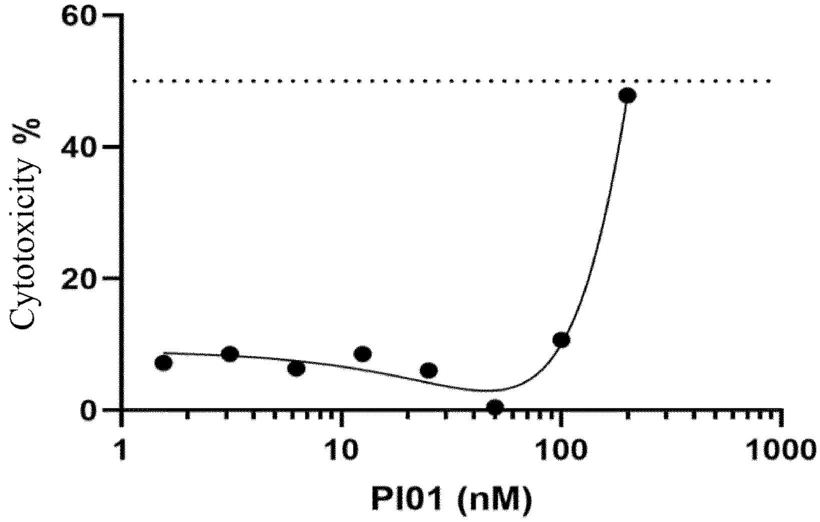
60
40
20
0
Cytotoxicity %
1
10
100
1000
PI01 (nM)

*FIG. 1A*

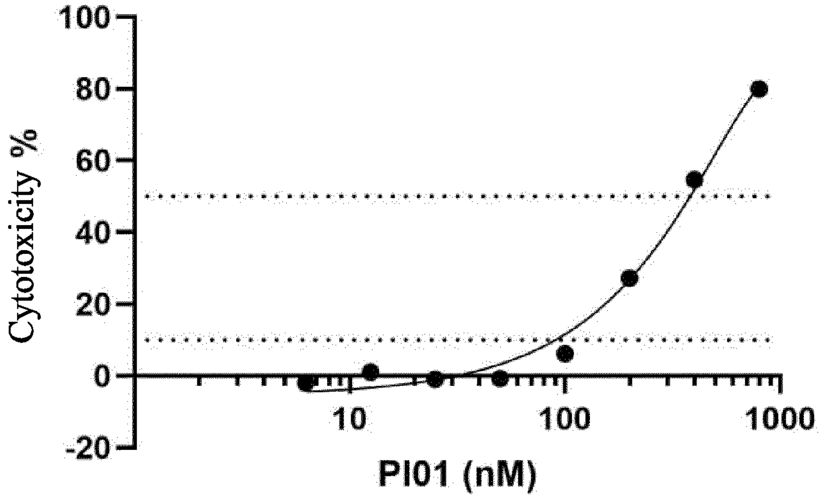
100
80
60
40
20
0
-20
Cytotoxicity %
10
100
1000
PI01 (nM)

*FIG. 1B*

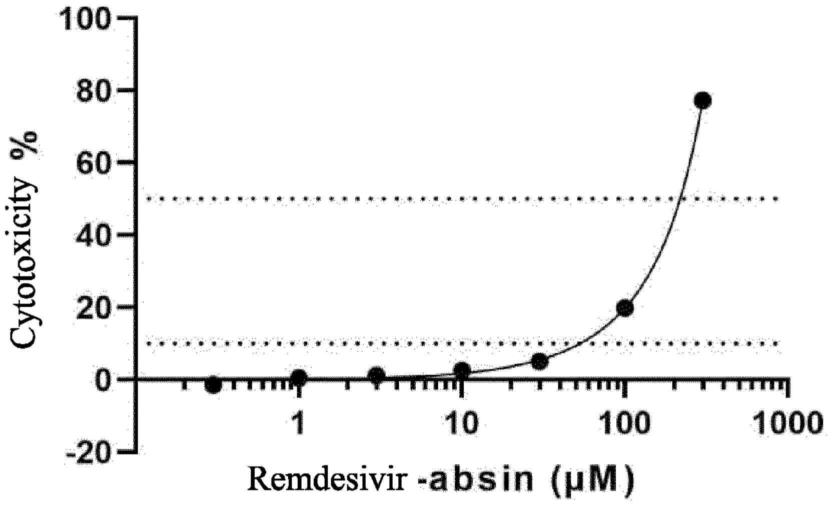
100
80
60
40
20
0
-20
Cytotoxicity %
1
10
100
1000
Remdesivir -absin (μM)

*FIG. 1C*

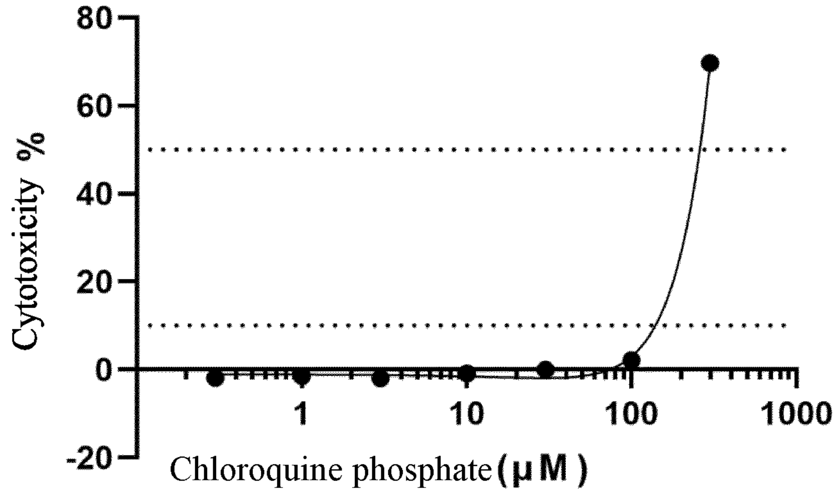
80
60
40
20
0
-20
Cytotoxicity %
1
10
100
1000
Chloroquine phosphate (μM)

*FIG. 1D*

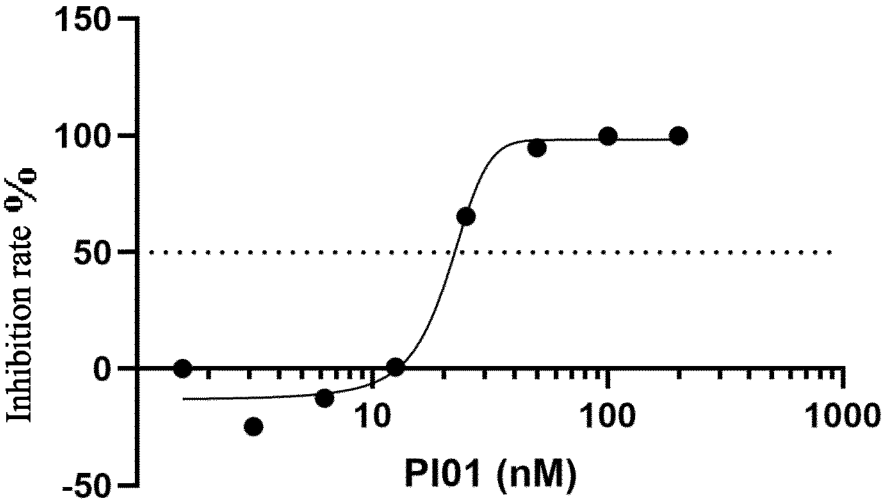
150
100
50
0
-50
Inhibition rate %
10
100
1000
PI01 (nM)

*FIG. 2A*

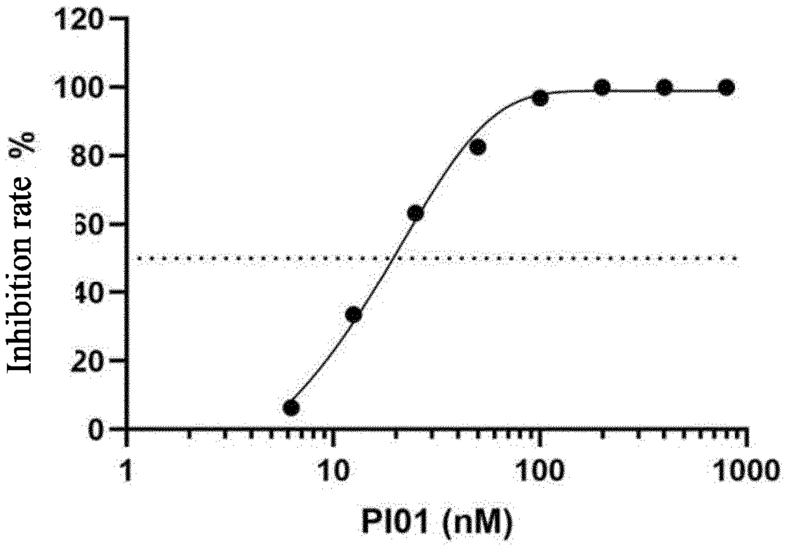
120
100
80
60
40
20
0
Inhibition rate %
1
10
100
1000
PI01 (nM)

*FIG. 2B*

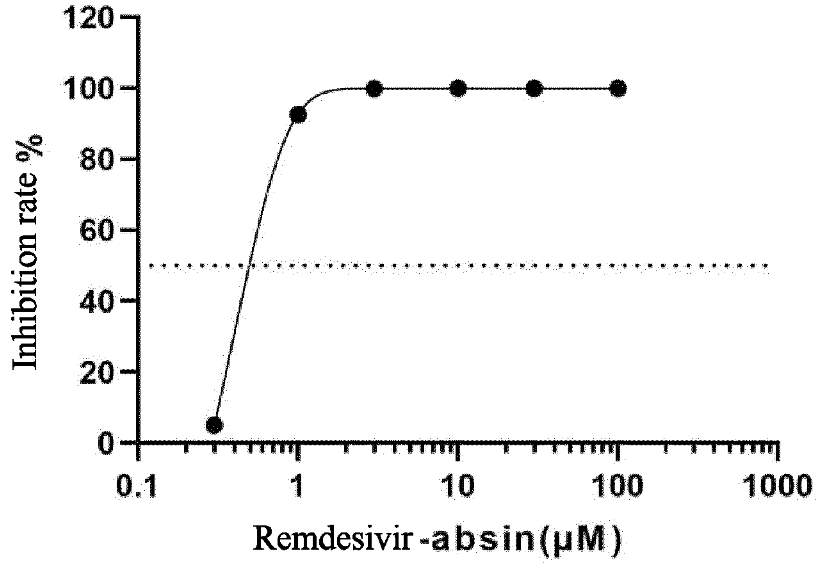
Inhibition rate %
120
100
80
60
40
20
0
0.1
1
10
100
1000
Remdesivir -absin(μM)

*FIG. 2C*

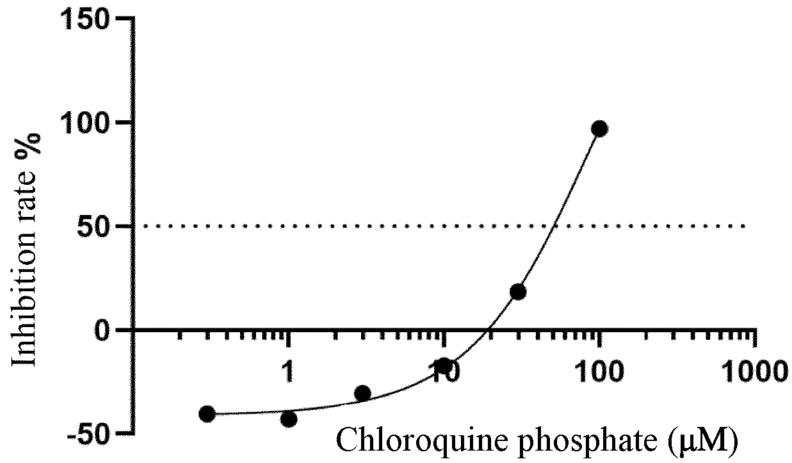
Inhibition rate %
150
100
50
0
-50
1
10
100
1000
Chloroquine phosphate (μM)

*FIG. 2D*

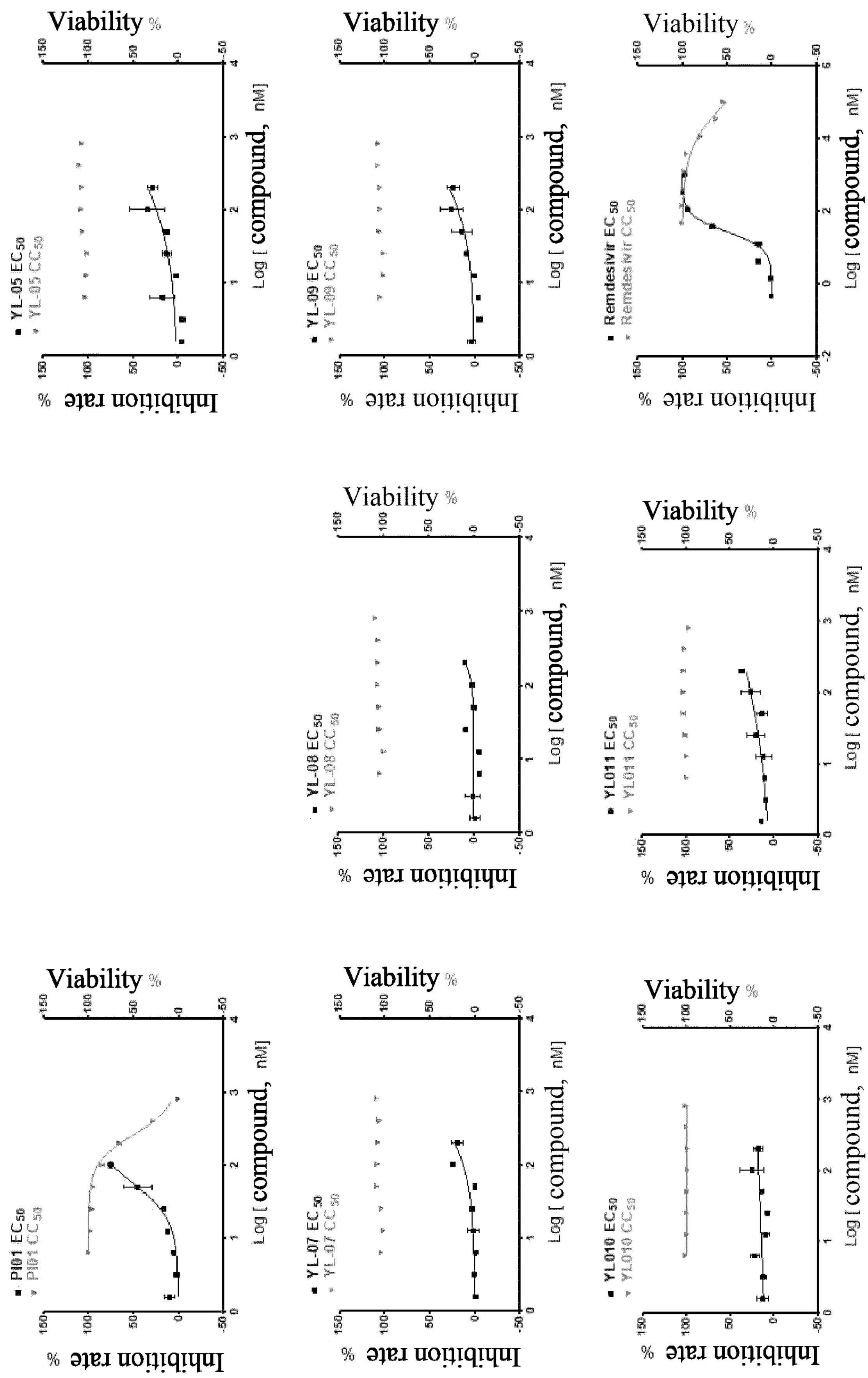
PI01 EC50
PI01 CC50
YL-05 EC50
YL-05 CC50
YL-07 EC50
YL-07 CC50
YL-08 EC50
YL-08 CC50
YL-09 EC50
YL-09 CC50
YL010 EC50
YL010 CC50
YL011 EC50
YL011 CC50
Remdesivir EC50
Remdesivir CC50
Viability %
Inhibition rate %
Log [ compound, nM]

*FIG. 3A*

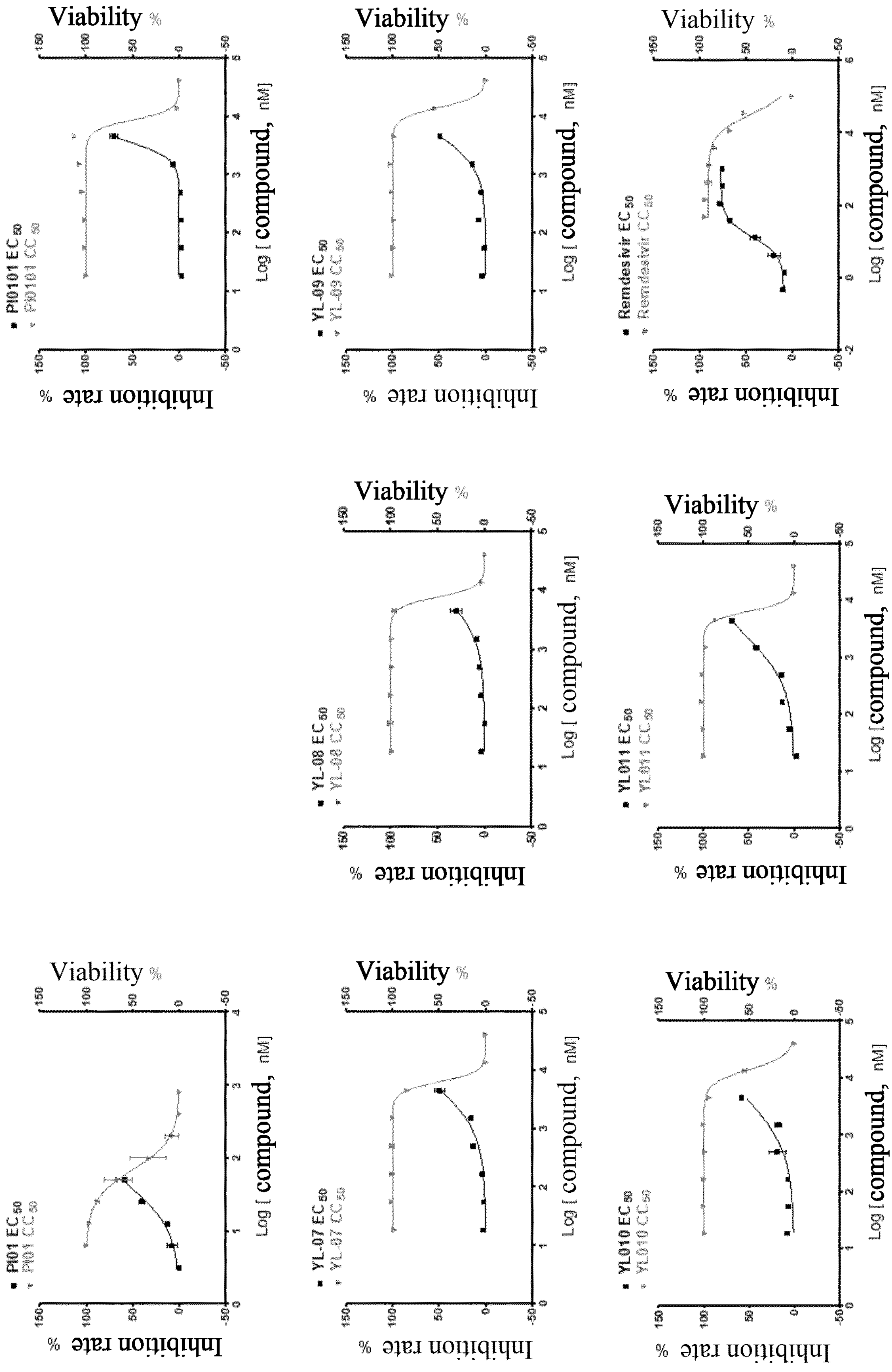
PI0101 EC50
PI0101 CC50
YL-09 EC50
YL-09 CC50
Remdesivir EC50
Remdesivir CC50
YL-08 EC50
YL-08 CC50
YL011 EC50
YL011 CC50
PI01 EC50
PI01 CC50
YL-07 EC50
YL-07 CC50
YL010 EC50
YL010 CC50
Viability %
Inhibition rate %
Log [ compound, nM]

*FIG. 3B*

**INTERNATIONAL SEARCH REPORT**

International application No.
**PCT/CN2021/080540**

**A. CLASSIFICATION OF SUBJECT MATTER**

A61K 45/00(2006.01)i; A61K 31/285(2006.01)i; A61K 31/555(2006.01)i; A61K 31/5377(2006.01)i; A61K 31/517(2006.01)i; A61P 31/14(2006.01)i; A61P 11/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; STNext: 挪贝肽; 冠状病毒; 氧化砷; 氧化苯砷; 司美匹韦; coronavirus; SARS; MERS; COVID-2019; Arsenic Oxide; oxyphenarsine; simeprevir

**C. DOCUMENTS CONSIDERED TO BE RELEVANT**

| Category* | Citation of document, with indication, where appropriate, of the relevant passages | Relevant to claim No. |
|---|---|---|
| PX | OLIVEIRA, Lima De et al. "Comparative Computational Study of SARS-CoV-2 Receptors Antagonists from Already Approved Drugs;" *ChemRxiv;*, 30 March 2020 (2020-03-30), Preprint. https://doi.org/10.26434/chemrxiv.12044538.v1; abstract | 9 |
| X | WO 2017147526 A1 (THE BOARD OF TRUSTEES OF TEH LELAND STANFORD JUNIOR UNIVERSITY et al.) 31 August 2017 (2017-08-31) claims 1, 11-16 | 1-8, 17-23 |
| Y | WO 9851297 A1 (UNIV. NEW MEXICO et al.) 19 November 1998 (1998-11-19) claims 1-14 | 1-25 |
| Y | CN 1144536 A (RESEARCH DEVELOPMENT FOUNDATION) 05 March 1997 (1997-03-05) claims 3, 18-19; description page 4 paragraph 2; figure 6; embodiment 10 | 1-25 |
| A | WO 2016173562 A1 (JIANGSU NUO-BETA PHARMACEUTICAL TECHNOLOGY CO., LTD.) 03 November 2016 (2016-11-03) claims 1-15 | 1-25 |
| A | WO 9107957 A1 (THE UNIVERSITY OF PITTSBURGH) 13 June 1991 (1991-06-13) claims 1-8 | 9-16, 24-25 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 May 2021** | **22 June 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/080540**

**C. DOCUMENTS CONSIDERED TO BE RELEVANT**

| Category* | Citation of document, with indication, where appropriate, of the relevant passages | Relevant to claim No. |
|---|---|---|
| A | CN 101433548 A (DING, Xinkan) 20 May 2009 (2009-05-20) entire document | 1-25 |
| A | WO 2012174312 A2 (GLAXOSMITHKLINE LLC. et al.) 20 December 2012 (2012-12-20) claims 1-70 | 9 |
| A | WO 2015135652 A1 (TECHNISCHE UNIVERSITAT MüNCHEN et al.) 17 September 2015 (2015-09-17) claim 11 | 9 |

Form PCT/ISA/210 (second sheet) (January 2015)

**INTERNATIONAL SEARCH REPORT**
**Information on patent family members**

International application No.
**PCT/CN2021/080540**

| Patent document cited in search report | Publication date (day/month/year) | Patent family member(s) | Publication date (day/month/year) |
|---|---|---|---|
| WO 2017147526 A1 | 31 August 2017 | US 2019062323 A1 | 28 February 2019 |
| | | EP 3419980 A4 | 03 July 2019 |
| | | EP 3419980 A1 | 02 January 2019 |
| WO 9851297 A1 | 19 November 1998 | CA 2290005 A1 | 19 November 1998 |
| | | US 6949665 B2 | 27 September 2005 |
| | | EP 0981344 A1 | 01 March 2000 |
| | | US 2002115713 A1 | 22 August 2002 |
| CN 1144536 A | 05 March 1997 | RU 2194754 C2 | 20 December 2002 |
| | | SA 714 B1 | 22 April 2006 |
| | | EP 0755440 A1 | 29 January 1997 |
| | | US 5532154 A | 02 July 1996 |
| | | FI 963717 A | 20 November 1996 |
| | | NZ 283575 A | 30 March 2001 |
| | | IL 112990 A | 30 November 1999 |
| | | JP H09510613 A | 28 October 1997 |
| | | CA 2186044 C | 23 August 2005 |
| | | FI 963717 A0 | 19 September 1996 |
| | | CA 2186044 A1 | 28 September 1995 |
| | | CN 1122105 C | 24 September 2003 |
| | | WO 9525788 A1 | 28 September 1995 |
| | | ZA 9502259 A | 20 September 1996 |
| | | KR 100394163 B1 | 20 October 2004 |
| | | AU 688887 B2 | 19 March 1998 |
| | | IL 112990 D0 | 29 June 1995 |
| | | ZA 952259 B | 20 September 1996 |
| | | AU 2191395 A | 09 October 1995 |
| | | JP 3821484 B2 | 13 September 2006 |
| | | KR 970701778 A | 12 April 1997 |
| | | EP 0755440 A4 | 19 August 1998 |
| WO 2016173562 A1 | 03 November 2016 | JP 2020203907 A | 24 December 2020 |
| | | JP 2018521112 A | 02 August 2018 |
| | | KR 102048137 B1 | 25 November 2019 |
| | | KR 20190131142 A | 25 November 2019 |
| | | RU 2017141805 A | 31 May 2019 |
| | | JP 6755938 B2 | 16 September 2020 |
| | | EP 3290514 A1 | 07 March 2018 |
| | | RU 2017141805 A3 | 31 May 2019 |
| | | RU 2724493 C2 | 23 June 2020 |
| | | KR 20180006396 A | 17 January 2018 |
| | | EP 3290514 A4 | 22 May 2019 |
| | | AU 2016255474 A1 | 21 December 2017 |
| | | CN 107849545 A | 27 March 2018 |
| | | US 2019314321 A1 | 17 October 2019 |
| | | WO 2016172952 A1 | 03 November 2016 |
| | | AU 2016255474 B2 | 30 January 2020 |
| | | AU 2020202847 A1 | 21 May 2020 |
| | | AU 2016255474 A8 | 01 March 2018 |
| WO 9107957 A1 | 13 June 1991 | US 4923895 A | 08 May 1990 |
| | | CA 2010651 A1 | 29 May 1991 |

Form PCT/ISA/210 (patent family annex) (January 2015)

**INTERNATIONAL SEARCH REPORT**
**Information on patent family members**

International application No.
**PCT/CN2021/080540**

| Patent document cited in search report | | | Publication date (day/month/year) | Patent family member(s) | | | Publication date (day/month/year) |
|---|---|---|---|---|---|---|---|
| CN | 101433548 | A | 20 May 2009 | WO | 2009121219 | A1 | 08 October 2009 |
| WO | 2012174312 | A2 | 20 December 2012 | WO | 2012174312 | A3 | 28 February 2013 |
| WO | 2015135652 | A1 | 17 September 2015 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MASTERS et al.** The molecular biology of coronaviruses. *Adv Virus Res.,* 2006, vol. 66, 193-292
- **HUANG et al.** Clinical features of patients infected with 2019 novel coronavirus in Wuhan, China. *Lancet,* 2020, 1-10
- **KIKKERT et al.** Innate Immune Evasion by Human Respiratory RNA Viruses. *Journal of Innate Immunity,* 2020, vol. 12 (1
- **XU et al.** Evolution of the novel coronavirus from the ongoing Wuhan outbreak and modeling of its spike protein for risk of human transmission. *SCIENCE CHINA Life Sciences,* 2020, vol. 63, 457-460
- **HUSTON JS et al.** *Proc Natl A cad Sci USA,* 1988, vol. 85, 5879
- **KWON J ; KIM D ; PARK J ; PARK Y ; HWANG Y ; WU H ; SHIN K ; KIM I.** Targeting Phosphatidylinositol 4-Kinase IIIa for Radiosensitization: A Potential Model of Drug Repositioning Using an Anti-Hepatitis C Viral Agent. *Int J Radiation Oncol Biol Phys,* 2016, vol. 96 (4), 867-876